# EUROPEAN PATENT APPLICATION

(11) **EP 4 793 361 A1**
(43) Date of publication of application: **19.08.2026**
(21) Application number: 24875834.4
(22) Date of filing: 09.10.2024
(51) Int. Cl.: C12Q 1/68, C12Q 1/6809, G01N 33/574, C12Q 1/6816, C12Q 1/6851, C12Q 1/686, C12Q 1/6886

(54) **IN VITRO DIAGNOSTIC AND/OR PROGNOSTIC PROCESS FOR TUMORS IN LIQUID SAMPLES, KIT AND IN VITRO CLINICAL MONITORING PROCESS**

(30) Priority: 09.10.2023 BR 102023020994; 03.06.2024 BR 102024011142
(71) Applicant: Liqsci Biotecnologia Ltda, 01310-200 São Paulo (BR)
(72) Inventor: REIS, Beatriz Da Costa Aguiar Alves, 05450-001 São Paulo (BR); VEIGA, Glaucia Raquel Luciano Da, 09060-050 Santo André (BR); FONSECA, Fernando Luiz Affonso, 04605-003 São Paulo (BR)
(74) Representative: WSL Patentanwälte Partnerschaft mbB
(86) International application number: PCT/BR2024/050465
(87) International publication number: WO 2025/076607

(57) **Abstract**

The present invention is in the field of Molecular Biology and Medicine. More specifically, the present invention discloses a kit and an *in vitro* process for tumor diagnosis and/or prognosis in liquid biological samples, being particularly useful in the diagnosis and/or prognosis of breast cancer. The process involves a step of quantifying *HIF-1α* gene expression and comparing it to a pre-determined level, optionally combining it with the expression level of other markers. In one embodiment, quantification is performed by dPCR. The present invention provides a new and improved cancer screening strategy, particularly for breast cancer, and is useful for both clinical and research purposes.

## Description

### Field of the Invention

The present invention is in the field of Molecular Biology and Medicine. More specifically, the present invention discloses a kit and an *in vitro* process for tumor diagnosis and/or prognosis in liquid biological samples, being particularly useful in the diagnosis and/or prognosis of breast cancer. The process involves a step of quantifying *HIF-1α* gene expression and comparing it to a predetermined level, optionally combining it with the expression level of other markers. In one embodiment, quantification is performed by dPCR. The present invention provides a new and improved cancer screening strategy, particularly for breast cancer, and is useful for both clinical and research purposes.

### Background of the Invention

Breast cancer is the leading cause of cancer-related deaths among women. According to INCA's estimate for the years 2023 to 2025, 73.6 new cases of breast cancer are expected in Brazil, with an estimated risk of 66.54 cases per 100,000 women ¹.

Tumor cells are capable of spreading to all parts of the body through the mechanisms of invasion and metastasis. Invasion refers to the direct penetration of tumor cells into adjacent tissues. Metastasis, in turn, refers to its ability to penetrate lymphatic and blood vessels, circulate through the bloodstream, and settle in other areas of the body, where it grows and forms colonies of the primary neoplasm ². Breast cancer is a clinically heterogeneous disease, and approximately 10-15% of patients show an aggressive disease and develop metastasis within the first three years after the primary tumor detection. In these patients, more than the primary tumor, distant metastases are the main cause of death ³.

This clinical heterogeneity (pathophysiology, progression, and response to treatment) of breast cancer is due to the fact that tumor development involves the interconnection of various signaling pathways, the cellular microenvironment, and the innate characteristics of each patient. In this context, the extracellular matrix (ECM) is one of the main components of the cellular microenvironment, being vital for the integrity and balance of tissues. Heparan sulfate (HS) plays a crucial role in the ECM, maintaining its structural integrity and regulatory functions through heparan sulfate proteoglycans (HSPG) ⁵⁸. Heparanase (HPSE) is the only enzyme capable of cleaving heparan sulfate, resulting in ECM remodeling and the release of growth factors and cytokines previously bound to HS. This, in turn, drives physiological and pathological processes such as angiogenesis, immune cell migration, inflammation, wound healing and metastasis ⁵⁹.

Furthermore, tumor progression (i.e., proliferation, invasion, and metastasis) requires a high bioenergetic expenditure ⁴. To meet energy needs, the molecules most used by cells are glucose and fatty acids. Furthermore, the most efficient cellular process for generating energy is the metabolism of glucose in the presence of oxygen. During this process, the products of glycolysis, such as pyruvate, are used in the citric acid cycle and in oxidative phosphorylation (OXPHOS) in the mitochondria, generating 38 ATP molecules/glucose ⁵.

Despite adequate glucose uptake, not all cells have access to a sufficient supply of oxygen to function properly. To grow beyond about 1 mm in diameter, tumors must form their own vascular network of blood supply, which happens through the influence of tumor angiogenesis factors ⁶. However, the new vascular network formed differs from that found in healthy tissues by presenting a series of structural and functional alterations that interfere with the adequate supply of blood and oxygen ⁴.

"Hypoxia" is the term used to describe oxygen deficiency in tissues. For most cells, hypoxia is characterized by molecular oxygen levels lower than 2%, with values around 1.2% characterizing moderate hypoxia and levels below 0.2% characterizing profound hypoxia or anoxia ⁷. To survive this condition, cells develop mechanisms to restore adequate tissue oxygenation or adapt to the new physiological conditions by converting to anaerobic metabolism. In the absence of oxygen, the pyruvate generated in the breakdown of glucose is not used by the mitochondria and, while still in the cytoplasm, is converted into lactate by means of fermentation. This process, however, is quite inefficient, since it generates a much smaller amount of energy (2 ATPs/glucose) ⁵. Thus, when comparing the efficiency of aerobic and anaerobic respiration processes, it is estimated that cells under hypoxia require 19 times greater glucose uptake than those under normoxia ⁸. Despite this, the strong selective pressure present in the tumor microenvironment leads to the selection of clones that can quickly generate energy (ATP) at the expense of efficiency, in addition to providing the necessary nutrients to dividing cells ⁹. Thus, hypoxia is a characteristic of most tumors ⁷.

The Warburg effect is known as aerobic glycolysis, in which glucose is converted into lactic acid even in the presence of oxygen. In this context, while some tumor cells exhibit oxidative phosphorylation, most of the glucose taken up by these cells (approximately 66%) is metabolized by fermentation, a process ten times faster than the complete oxidation of this molecule ⁸. Despite the apparent waste of glucose, aerobic glycolysis brings benefits to tumor cells, since it allows the fast generation of ATP, fatty acids and nucleic acids as long as glucose is abundant and ensures the creation of a tumor environment protected from attacks by the immune system, thanks to the excess production of lactic acid ^{5,10}. Furthermore, a glucose flow model indicates that even in healthy cells the mere presence of high amounts of this sugar causes the cell to "prefer" the anaerobic glycolysis pathway to the oxidative phosphorylation pathway, suggesting the presence of a cytoplasmic glucose sensor ¹¹. The flow of glucose and its intracellular concentration in tumor cells could, therefore, be the trigger for the Warburg effect.

Given the above, increased glycolysis is seen as an essential component of tumor malignancy and is considered a hallmark of invasive tumors ⁸. In fact, the presence of hypoxic regions in solid tumors is an indicator of a worse prognosis, since hypoxia contributes to increased aggressiveness of the malignant transformation process and protects the tumor against conventional treatment methods ⁷.

The effects of cellular oxygen sensors are regulated by Hypoxia-Inducible Factors (HIF), a highly conserved family of transcription factors activated by hypoxia ¹². In their active form, HIFs comprise a heterodimer with a subunit α (HIF-1α, HIF-2α, or HIF-3α) and a β subunit (HIF-1β, also called ARNT - aryl hydrocarbon nuclear receptor translocator). HIF-1 activation under hypoxia depends on its subunit α, which is sensitive to oxygen levels. The other subunit of HIF-1 (HIF-1β) is constitutively expressed and only binds to HIF-1α when its levels are increased ¹². Under normal oxygen tension conditions, HIF-1α is hydroxylated by the action of the enzyme propyl hydroxylase; in this case, the von Hippel-Lindau (VHL) tumor suppressor protein recognizes hydroxylated HIF-1α and degrades it by ubiquitination. Under hypoxic conditions, however, the hydroxylation rate drops, causing HIF-1α to no longer be recognized by the VHL protein, leading to its stabilization and overexpression ¹³. Once stable, HIF-1α is transported to the nucleus, where it binds to the β subunit; the heterodimer formed binds to DNA via the recognition sequence (5'_RCGTG_3') present in the hypoxia-response elements (HRE) of several genes that will stimulate the tumor process, such as angiogenesis, glycolysis, erythropoiesis and, if they fail, apoptosis ¹⁷. For example, once activated, HIF1s modulate the expression of genes that encode proteins in the glycolytic pathway (enzymes and transporters) such as hexokinase (HK), pyruvate kinase (PK), and lactate dehydrogenase (LDHA) ¹⁸. Alterations in glycolytic proteins expression mediated by HIF-1a lead to metabolic reprogramming of the tumor cell that not only allows it to survive hypoxic conditions ¹⁹, but also makes it resistant to chemo- and radiotherapy treatments ²⁰.

Clinically, hypoxia and its inducible factors (HIF-1 and HIF-2) are associated with increased distant metastases and a worse prognosis in a number of tumors ²¹. To date, it is known that increased expression of HIF-1 (HIF-1α and HIF-1β complex) is present in 53% of all types of cancer, and in 56-76% of breast cancers ⁷, and that the activity of this protein is one of the determining factors in tumor development and is associated with invasion, metastasis, and prognosis ²². Analyzing samples of breast tumors, Bos et al. ²³ demonstrated that HIF-1 expression is higher in samples with a more advanced stage, a result that positively associates this factor with tumor aggressiveness. He et al. ²², in turn, working with pancreatic tumor cells, observed that under hypoxic conditions, the proteins PKM2, PDK1, and LDHA were overexpressed; furthermore, the suppression of HIF-1α by knockdown in these cells caused the production of lactate to be reduced and the expression of said enzymes to be blocked, leading to the repression of glycolysis.

In addition to activating the production of enzymes in the glycolytic pathway, another gene whose expression is modulated by HIF-1α during tissue hypoxia is Leukemia Inhibitory Factor (LIF), expressed at high levels in various malignant cells from different tissues ^{49,50}. LIF is a glycoprotein cytokine belonging to the neuropoietic cytokine family, which includes, for example, interleukin-6 (IL-6), oncostatin M (OSM), and IL-11 ^{51,52}. It is a multifunctional protein capable of acting in various systems and tissues ^{49,51}. This action is based on the regulation of cell survival and proliferation through the activation of different molecular pathways, mainly JAK/STAT ⁴⁹⁻⁵³. LIF performs its function by binding to a heterodimeric receptor formed by LIFR (LIF receptor) and gp130 (glycoprotein 130) ⁵⁴. According to the authors, LIF plays a role in tumor promotion, while LIFR acts both as a tumor promoter and repressor. Thus, physiologically, this molecule signals cellular processes that maintain the self-renewal and pluripotency of embryonic stem cells, potentially acting both as a stimulating and inhibiting factor, leading to the proliferation or differentiation depending on the cells involved ⁴⁹.

Although several studies have demonstrated an association between high LIF levels and tumor promotion, recent studies seem to suggest a potentially paradoxical relationship where high LIF expression could be associated with a favorable outcome ⁶⁰.

The document "Johnson et al., Induction of LIFR confers a dormancy phenotype in breast cancer cells disseminated to the bone marrow, Nat Cell Biol. 2016" reveals the influence of LIFR on the invasive or dormant phenotype of breast cancer cells that have spread to the bones. According to the authors, LIFR:STAT3:SOCS3 signaling in disseminated breast cancer cells confers a dormant phenotype through the maintenance of STAT3 signaling in response to LIF. This document does not mention or suggest the invention disclosed herein.

The document "lorns et al., Whole genome in vivo RNAi screening identifies the leukemia inhibitory factor receptor as a novel breast tumor suppressor, Breast Cancer Res Treat. 2012" reveals that HMLE cells were transduced with a library containing RNA interference targeting the entire human genome and xenografted into the mammary fat layers of mice to identify human breast tumor suppressor genes. This study identified LIFR as a tumor suppressor. This document does not mention or suggest the invention disclosed herein.

The document "Chen et al., LIFR is a breast cancer metastasis suppressor upstream of the Hippo-YAP pathway and a prognostic marker. Nat Med. 2012" reveals a study on the protein expression of LIFR and E-cadherin in human breast cancer cell lines. According to the authors, LIFR expression suppresses both the early (invasion) and late (colonization) stages of metastasis. The loss of LIFR triggers migration, invasion, and metastatic colonization of breast cancer cells through the activation of YES-associated protein (YAP). This document does not mention or suggest the invention disclosed herein.

### Screening and monitoring methods

Currently, breast cancer screening is performed using imaging tests, such as ultrasound or mammography. According to INCA ²⁴, despite the benefits of early detection of breast cancer, mammography also has drawbacks, such as the generation of incorrect results (false positives or false negatives), since, as it is an imaging exam, it depends on the correct training of the technician performing the exam, as well as the proper maintenance of the equipment. Three decades after the start of the use of mammography as a breast cancer screening technique, early detection of the disease has been accompanied by an increase in the incidence of tumors in early stages with low-risk biological characteristics and an increase in the detection of in situ disease, without, however, a concomitant decrease in the incidence of tumors in more advanced stages ²⁵.

In Brazil, breast cancer screening is carried out via primary care by the Brazilian Unified Health System *(SUS)* ²⁶. The Ministry of Health recommends that screening mammograms be performed biennially only in women over 50 years of age, since before that age dense breasts make it difficult to accurately analyze the results. Although the incidence of breast cancer is considerably higher in women over 50 years old, in women under 35 it has increased from 2% to 5% in recent years ²⁷, and more than 40% of breast cancer cases in Brazil are diagnosed in women under 50 years old ²⁸. In young women, breast cancer is known to tend to be more aggressive ²⁹ and is only identified after the appearance of lumps or suspicious changes in the breasts. Other difficulties encountered by patients include obtaining an accurate prognosis. Approximately 70% of patients with early-stage breast cancer receive adjuvant chemotherapy, but only a specific group truly benefits from this treatment ⁵⁵. These data point to the need for complementary methods of breast cancer screening and early detection, as well as methods for monitoring and predicting clinical outcomes that are universally applicable and, preferably, low-cost.

In this context, biopsy of the affected tissue is the gold standard method for diagnosing neoplasia and can also be used in genomic platforms to obtain a prognosis. Among these platforms, *Oncotype* DX^{® 56} stands out, analyzing the expression of a set of 21 genes related to cell proliferation, metastatic capacity, and hormone expression, capable of predicting overall survival and recurrence-free survival. The prognostic validity of the score obtained through this genomic analysis has already been confirmed in several patient populations ⁵⁷. Thus, the *Oncotype* test allows for a prognostic assessment of the patient through the genetic profile of the primary breast tumor.

However, tissue biopsy is an invasive method which is not free from complications, such as bleeding, infection and pain; furthermore, it depends on the accessibility of the lesion and the collection of adequate quantities, since tumors are heterogeneous ³⁰. This is one of the reasons why complementary alternatives for cancer diagnosis and monitoring are still needed. One of the alternative methods is liquid biopsy, a minimally invasive technique that is easily repeated over time and analyzes samples of non-solid biological tissues, such as peripheral blood, urine, saliva, cerebrospinal fluid, and pleural fluid. The fluids commonly used in liquid biopsy present circulating tumor DNA (ctDNA), circulating tumor RNA (ctRNA), proteins, exosomes, and circulating tumor cells (CTC) as biomarkers, thus allowing the identification of a specific molecular profile for each patient ³².

In solid tumors (such as breast cancer), tumor cells can spread from the primary tumor during its progression. These cells can remain in the body after tumor removal and are currently undetectable by conventional diagnostic methods, which characterizes minimal residual disease (MRD) ^{33,34}. These circulating tumor cells (CTC) existing in patients peripheral blood have attracted the attention of the medical community since the analysis of mutations or gene expression in these cells can be a valuable tool for monitoring the tumor throughout treatment ³⁵. In breast cancer patients, the detection of CTC in peripheral blood has been associated with reduced overall and progression-free survival ³⁶⁻³⁸. Since CTC are highly heterogeneous, their molecular characterization through the expression study of different genes is of crucial importance not only to confirm their origin, but also to detect phenotypic changes associated with tumor progression and resistance to chemotherapy treatment.

The document "Peiró et al., Diagnostic potential of hypoxia induced genes in liquid biopsies of breast cancer patients, Sci Rep 11, 2021" reveals that HIF-1α transcripts can be found in peripheral blood of women affected by breast cancer, as well as in healthy women. However, the expression of this gene is significantly higher in samples from breast cancer patients than in those from healthy women ³⁹. It is worth noting that *HIF-1α* is detected in cancer-negative blood samples, as this gene is also important for several functions under physiologically normal conditions. For example, HIF-1α is expressed on CD4⁺ cells in both hypoxia and normoxia ⁴³ and on various types of cells of the innate immune system, such as macrophages, dendritic cells, neutrophils and Th17 cells ⁴². Furthermore, HIF-1α is a component of pathways involved in the control of cellular metabolism, playing a role in regulating the effector functions of immune system cells ⁴². According to the authors, HIF-1α is critical for the maturation of dendritic cells and for the activation of T cells.

Additionally, it is noteworthy that the document of Peiró et al (2021) uses the real-time PCR (qPCR) method to evaluate the expression of *HIF-1α.* Although this is the most commonly used technique for evaluating gene expression, the results generated can vary considerably depending on inadequate dilutions, low cellularity of the samples, or chemical contaminants that inhibit Taq Polymerase and the hybridization of primers and probes ⁴⁴. Due to pre-analytical and technical limitations, samples with low cellularity are generally discarded from analyses ⁴⁵.

In this context, the development of the digital PCR (dPCR) platform has enabled the expansion of PCR technology for diagnosis and research purposes ⁴⁶. The principle of digital PCR, as with all technologies based on PCR, is the specific amplification of a target consisting of nucleic acid ⁴⁷. What differentiates dPCR from others is the fact that the reagents are separated into hundreds of thousands of partitions or bubbles, in which amplification reactions occur in real time. The distribution of target sequences in these partitions or bubbles is described by the Poisson distribution, which allows for their absolute and precise quantification based on the rate of positive partitions against all amplified partitions. This analysis eliminates the need for a reference target with a previously known concentration and increases the accuracy of quantifying rare or low-concentration targets when compared to qPCR ⁴⁸. Thus, while the result generated by qPCR depends on a well-established standard curve, dPCR generates results in copies/µL, that is, in absolute numbers that are easy to interpret. Furthermore, dPCR technology has proven to be less affected by contaminants or inhibitors found in different types of biological samples. Thus, for the detection of low-concentration targets (Number of cycles (Cq) ≥ 29) and/or with chemical contaminants and proteins, dPCR generates more accurate, reproducible and statistically significant results ⁴⁴. Falzone et al. ⁴⁵ compared the detection efficacy of qPCR and dPCR for low-viral load samples. According to the authors, qPCR with intercalating fluorophores was not efficient in this detection, while the use of hydrolysis probes resulted in these samples being detected with late Cqs using SYBr-Green. Using dPCR, low-viral load samples were detected as positive at dilutions of up to 10 times.

In addition to the articles referenced above, the following documents related to the topic were found when searching the prior art in patent literature:

CN103608028 discloses a method for cancer detection and monitoring, in which the presence of myeloid-derived suppressor cells (MDSC) is indicative of cancer. The increased expression of the HIF1α, STAT3, and C/EBPβ genes distinguishes MDSC from normal myeloid cells. Furthermore, the phenotype is evaluated by flow cytometry, and gene expression can also be assessed by qPCR. This document does not disclose the subject matter claimed herein.

WO2018150031 discloses a method for detecting breast cancer using selected liquid samples of blood and/or bone marrow aspirate. More specifically, this method focuses on the detection of circulating tumor cells (CTC) by ELISA analysis of a protein expressed exclusively in tumor cells, Cyr61. Additionally, this method may involve the simultaneous detection of Cyr61 with HIF-1 and/or PD-L1. It is worth noting that the method disclosed in WO2018150031 presents several drawbacks to its applicability, such as: low screening scope, given that not all patients with solid tumors have CTC; low effectiveness for early detection, since the presence of CTC indicates a more advanced stage of the neoplasm; and it is expensive, since it requires the separation of CTC from the sample.

The present invention is an alternative to known strategies for screening and monitoring tumors.

Thus, based on the literature reviewed, no documents were found that anticipated or suggested the teachings of the present invention, so the solution proposed herein, in the eyes of the inventors, possesses novelty and inventive activity compared to the prior art.

### Summary of the Invention

The present invention solves the problems of the prior art by providing an *in vitro* process for cancer screening, diagnosis and/or prognosis comprising the quantification of *HIF-1α* gene expression and, optionally, other markers such as *LIF* in body fluids or liquid biological samples. In one embodiment, said quantification is performed by dPCR.

The present invention provides an effective, accessible, and minimally invasive way to verify the presence or absence of tumor indicators. The invention is particularly useful in the screening, diagnosis, and monitoring of breast cancer, providing the assessment of markers expression in the blood as a useful laboratory tool to complement cancer screening, diagnosis and/or prognosis, as well as serving for the follow-up of patients and women with a family history of this neoplasm.

The process and kit of the invention provide remarkable improvements in the diagnostic utility, in liquid biological samples, for detecting the presence of tumors through the quantitative assessment of *HIF-1α* gene expression from the quantification of the respective mRNA by dPCR. This improvement is particularly noticeable in the case of breast cancer diagnosis.

The process and kit of the invention have prognostic utility, regardless of the amplification approach (whether qPCR or dPCR) when performed: (i) quantitative assessment of *HIF-1α* gene expression based on the quantification of the respective mRNA present in a liquid biological sample and its normalization by comparison with a reference gene expression; and (ii) quantitative assessment of the expression of another gene selected from *MCT1, MCT4, CD147, GLUT1, LIF,* based on the quantification of the respective mRNA present in a liquid biological sample and its normalization by comparison with a reference gene expression; (iii) the correlation between quantification of *HIF-1α* expression, expression quantification of said other gene, or between the proportion between these latter, with tumor prognosis.

In a first aspect, the present invention provides an *in vitro* process for tumor screening, diagnosis and/or prognosis comprising the steps of:
a) preparing an analyte from a liquid sample;
b) quantifying the expression of *HIF-1α* from the total RNA in the sample;
c) optionally, normalizing the quantification of *HIF-1α* expression from the quantification of a reference marker; and
d) comparing the quantification of *HIF1α* expression with a predetermined level, indicating the presence of a tumor when the value is above said level, or the absence of a tumor when the value is below said level.

In a second aspect, the present invention provides a kit for tumor screening, diagnosis and/or prognosis comprising:
- reagents for stabilizing the total RNA in the liquid sample;
- enzyme to enable the formation of cDNA from the mRNA of the HIF1α gene and, optionally, from the mRNA of a reference marker gene;
- primers, probes, dNTPs for amplification of *HIF-1α* cDNA and, optionally, of the reference marker gene cDNA; and
- reagents for the quantitative visualization of amplified cDNAs.

Non-limiting examples of reference marker genes include *RPL13a, GUSB, TRFC,* and *Beta-actin.*

In a third object, the present invention provides an *in vitro* process for monitoring the clinical status of an individual with breast cancer under treatment, comprising the steps of:
a) quantifying *LIF* expression in a liquid sample from an individual with breast cancer under treatment;
b) quantifying *LIF* expression in a liquid sample from an individual with breast cancer not under treatment;
c) comparing the quantification of *LIF* expression in the sample from the individual with breast cancer under treatment to the quantification in the sample from the individual with breast cancer not under treatment,
wherein *LIF* expression values in the sample from the individual with breast cancer under treatment that are higher than those in the sample from the individual with breast cancer not under treatment indicate an improvement in the clinical status.

These and other objects of the invention will be immediately appreciated by those skilled in the art and will be described in detail below.

### Brief Description of the Figures

The following figures are presented:
Fig. 1 shows a visualization of fluorescence detection in the 26,000 partitions of the dPCR plate. Item A refers to the sample of a healthy woman; Item B refers to the sample of a woman with breast cancer.
Fig. 2 shows a graphical visualization of the dPCR result. Positive partitions (above the lines) exhibit fluorescence emission greater than negative partitions (below the lines).
Fig. 3 shows a graphical representation of the difference in the number of transcript copies/µL of *HIF-1α* in healthy women (CTL) and women with breast cancer (BC).
Fig. 4 shows the ROC curve analysis for evaluating the accuracy of the absolute determination of the number of HIF-*1α* transcript copies in peripheral blood as a diagnostic breast cancer marker in liquid biopsy.
Fig. 5 shows a graphical representation of the difference in the number of transcript copies/µL of *HIF-1α* in healthy women and women with breast cancer (BC) obtained with a larger sample size.
Fig. 6 shows the ROC curve analysis for evaluating the accuracy of the absolute determination of the number of copies of *HIF-1α* transcripts normalized by the number of copies of the *TRFC* reference gene in peripheral blood as a diagnostic breast cancer marker in liquid biopsy.
Fig. 7 shows a decision tree that incorporates all possible applications of the present invention in the various clinical scenarios of the patients.
Fig. 8 shows a graphical representation of *LIF* gene expression in peripheral blood samples from healthy donors and breast cancer patients (Mann-Whitney test, with a 95% confidence interval) with p = 0.1978. Gene expression was calculated using data at the time of diagnosis.
Fig. 9 shows a graphical representation of *LIF* gene expression in peripheral blood samples considering its distribution according to the clinical stage of breast cancer patients (95% CI) and p < 0.05. Gene expression was assessed using data collected at the time of diagnosis.
Fig. 10 shows a graphical representation of *LIF* gene expression in serial peripheral blood samples (collection 1 at the time of diagnosis; collection 2 after 3 months of treatment; and collection 3 after 6 months of treatment) from peripheral blood samples of breast cancer patients with negative results (item A) or positive progression (item B).
Fig. 11 shows a graphical representation of the correlation between the expression of *LIF* and *HIF-1α* genes in peripheral blood samples at the time of diagnosis (item A), 3 months after the start of chemotherapy treatment (item B), and 6 months after the start of chemotherapy treatment (item C) in breast cancer patients. 95% confidence interval, p < 0.0001 and r = - 0.4788 (item A), r = - 0.4774 (item B) and r = - 0.4734 (item C). Both *LIF* gene expression and *HIF-1α* gene expression were obtained using formula (I).
Fig. 12 shows a graphical representation of the correlation between *LIF* gene expression and heparanase quantification in peripheral blood samples from breast cancer patients: item A) at the time of diagnosis (95% confidence interval, p = 0.0006, r = -0.3180), item B) 3 months after the start of chemotherapy, and item C) 6 months after the start of chemotherapy. *LIF* gene expression was assessed using diagnostic data.
Fig. 13 shows a graphical representation of the correlation between *LIF* gene expression and *Oncotype* score in peripheral blood samples from breast cancer patients (95% confidence interval), p = 0.0186 and r = -0.2201. *LIF* gene expression was assessed using diagnostic data.

### Detailed Description of the Invention

The present invention, in all its embodiments, provides, among other technical advantages, the effective, accessible and less invasive screening, diagnosis and/or prognosis of tumors. The invention is particularly useful for the diagnosis and/or prognosis of breast cancer.

In the present invention, *HIF-1α* is used as a screening biomarker, optionally combined with other genes such as *LIF.*

The present invention provides an *in vitro* process for tumor screening, diagnosis and/or prognosis comprising the steps of:
a) preparing an analyte from a liquid sample;
b) quantifying the expression of *HIF-1α* from the total RNA in the sample;
c) optionally, normalizing the quantification of *HIF-1α* expression from the quantification of a reference marker; and
d) comparing the quantification of the normalized *HIF-1α* expression to a predetermined level, indicating the presence of a tumor when the value is above said level, or the absence of a tumor when the value is below said level.

In one embodiment, said *in vitro* process additionally comprises a step of quantifying the expression of one or more genes among: *GLUT1;* monocarboxylate transporters *MCT;* MCT chaperone *CD147;* Leukemia Inhibitory Factor *LIF.*

In one embodiment of said *in vitro* process, the liquid sample is blood and the quantification of total RNA is performed by dPCR.

In one embodiment of said *in vitro* process, step b) comprises the synthesis of cDNA from 100 ng of total RNA and the dilution of the synthesized cDNA 25 times or 50 times.

In one embodiment, step b) further comprises the amplification of *HIF-1α* cDNA, where the primers/probes for *HIF1α* cDNA amplification are selected from the following nucleotide sequences: Seq ID No.1; Seq ID No. 2; Seq ID No. 3, or combinations thereof.

In one embodiment, said normalization step is performed by comparison with the expression level of *RPL13a, GUSB, TRFC,* and/or *β-actin.*

In one embodiment of the *in vitro* process, said comparison step d) is performed by analyzing the Receptor Operating Characteristic (ROC) curve.

In one embodiment of the *in vitro* process, *HIF-1α* expression values above 5,010 transcript copies/µL indicate the presence of a tumor, and values below 5,010 transcript copies/µL indicate the absence of a tumor.

The *in vitro* process of the invention can be combined with other complementary tests to verify indications of tumors in patient samples.

The present invention also provides a kit for tumor screening, diagnosis and/or prognosis comprising:
- reagents for stabilizing the total RNA in the liquid sample;
- enzyme to enable the formation of cDNA from the mRNA of the *HIF-1α* gene and, optionally, from the mRNA of a reference marker gene;
- primers, probes, dNTPs for amplification of *HIF-1α* cDNA and, optionally, of the reference marker gene cDNA; and
- reagents for the quantitative visualization of amplified cDNAs.

In one embodiment, said primers/probes for *HIF-1α* cDNA amplification are selected from the following nucleotide sequences: Seq ID No.1; Seq ID No. 2; Seq ID No. 3, or combinations thereof.

In one embodiment, the kit additionally comprises primers and probes for the amplification of cDNA from one or more genes among: *GLUT1;* monocarboxylate transporters *MCT;* MCT chaperone *CD147;* Leukemia Inhibitory Factor *LIF.*

In one embodiment, the kit additionally comprises primers and probes for the amplification of cDNA from one or more reference genes among *RPL13a, GUSB, TRFC,* and/or *Beta-actin.*

In one embodiment, gene expression is quantified using the dPCR technique. For this purpose, total mRNA is extracted from liquid biological samples, preferably blood, to produce cDNA. The use of probes during dPCR makes the test more specific, avoiding false positives. Furthermore, the quantification process used herein allows for multiplex reactions, meaning that two or more transcripts can be amplified in the same sample. As a result, the process is optimized, with reduced time and cost for testing.

The present invention also provides an *in vitro* process for monitoring the clinical status of an individual with breast cancer under treatment, comprising the steps of:
a) quantifying *LIF* expression in a liquid sample from an individual with breast cancer under treatment;
b) quantifying *LIF* expression in a liquid sample from an individual with breast cancer not under treatment;
c) comparing the quantification of *LIF* expression in the sample from the individual with breast cancer under treatment to the quantification in the sample from the individual with breast cancer not under treatment,
wherein *LIF* expression values in the sample from the individual with breast cancer under treatment that are higher than those in the sample from the individual with breast cancer not under treatment indicate an improvement in the clinical status.

Unlike mammography, where women under 50 are more difficult to assess due to breast density, the process described herein is universally applicable since it refers to the *in vitro* analysis of liquid biological samples. Furthermore, since the process of the present invention utilizes the total RNA of the sample, it offers broad scope for tumor screening. Indeed, results for the use of HIF-1*α* as a breast cancer biomarker are surprising, with excellent performance evidenced by the high area under the curve (AUC) value. Thus, the process disclosed herein can provide transcript values that indicate the presence of disease in the biological sample before it can be detected by routine imaging tests.

In one embodiment, *HIF-1α* expression values above the limit determined by the Receptor Operating Characteristic (ROC) curve analysis are indicative of a tumor in the analyzed sample.

In the context of the present invention, one or more markers are selected from glucose transporters, preferably *GLUT1;* monocarboxylate transporters, preferably *MCT1* and/or *MCT4;* MCT chaperone *CD147;* carbonic anhydrases, preferably *CAIX;* mitogen- and stress-activated protein kinases MSK, preferably *MSK-1;* MIDKINE, Leukemia Inhibitory Factor *LIF,* among others.

### Definitions

The terms "liquid biological sample(s)" and "liquid sample(s)" refer to any non-solid sample or bodily fluid. Non-limiting examples include: peripheral blood, plasma, serum, saliva, urine, among others.

The term "tumor" or "tumors" refers to the abnormal proliferation of a tissue, which partially or totally escapes the control of the organism and tends towards autonomy and perpetuation; it can be a benign or malignant tumor. Preferably, tumor refers to breast cancer.

Unlike mammography and tissue biopsy, the ease and practicality of the process disclosed herein allows for its large-scale application, contributing to the early detection and monitoring of tumors, preferably breast cancer, in an effective and low-cost manner. Therefore, this process represents an alternative diagnostic and/or prognostic strategy that can contribute to the development of new public policies for the management of breast cancer.

### Examples

The examples shown herein are intended only to illustrate some of the numerous ways of carrying out the invention, without, however, limiting its scope.

### Example 1 - In vitro process for cancer diagnosis: quantification of HIF-1α in liquid biopsy using dPCR

To verify the potential of using the subunit *α* of Hypoxia-Inducible Factors (*HIF-1α*) for tumor screening and diagnosis, a study was conducted with healthy women and women diagnosed with breast cancer, using peripheral blood samples and dPCR to quantify the expression of the gene of interest.

In this example, blood samples from 22 healthy women and 16 cancer patients were included. The cDNA was synthesized from 100 ng of total RNA and diluted 50X. The amplification reactions were performed in a QlAcuity One Digital PCR System thermocycler (Qiagen cat no. 911021) using plates with 26,000 partitions per sample (Qiagen QlAcuity Nanoplate 26k 24-well, cat no. 250001) in a final volume of 40 µL, containing: 1X EvaGreen (QIAcuity^{®} EG PCR Kit, Qiagen cat. no. 250112) or 1x Probe PCR Kit (QlAcuity Probe PCR kit, Qiagen cat. nos. 250102, 250101, 250103), 15 pmol of each specific primer (table 1) and 2.0 µL cDNA. The thermocyclic parameters consist of an initial hot start step at 95°C for 2 minutes, followed by 40 repetitions of 95°C for 15 seconds, 60°C for 30 seconds, and 72°C for 15 seconds, ending with a 5-minute cooling step at 40°C. Fluorescence was captured by an exposure of 200 ms, with a gain value of 2.

**Table 1 - Characteristics of specific primers and probes and their amplicons.**

| Gene | SEQUENCE (5'-3') | AMPLICON (bp) |
|---|---|---|
| | F - SEQ ID NO:1 | |
| HIF-1α | R - SEQ ID NO:2 | 227 |
| | P - SEQ ID NO:3 | |

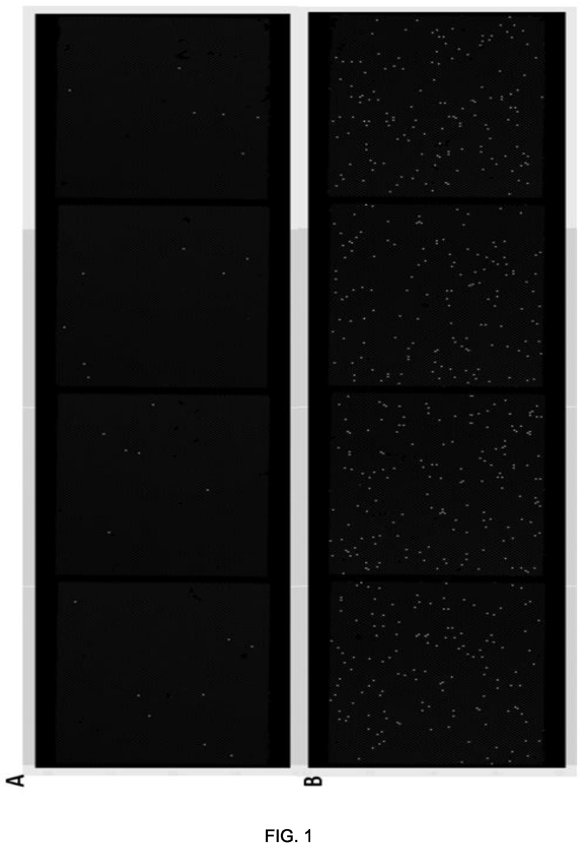

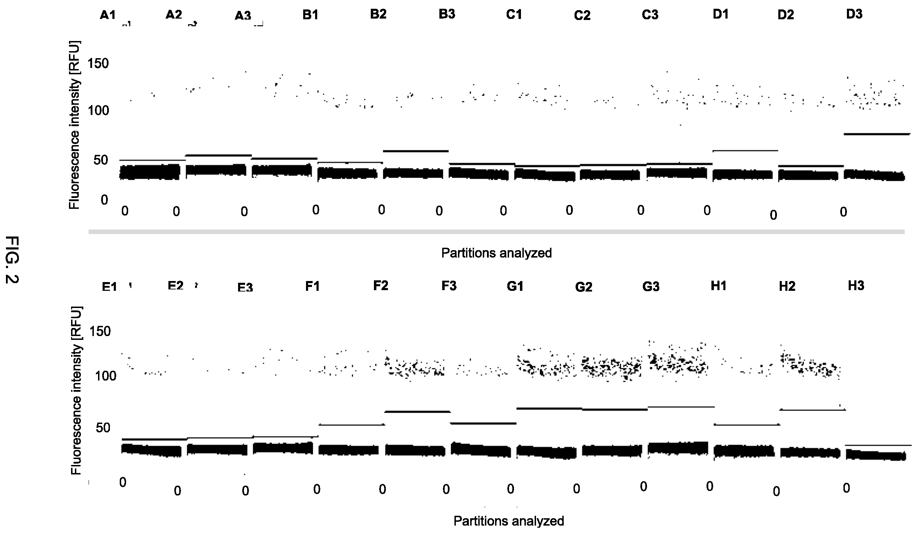

The absolute number of *HIF-1α* transcripts was assessed by the fluorescence intensity detected in each of these 26,000 partitions. Fig. 1 shows that the number of positive partitions in the healthy woman (item a) is lower than that found in the patient with breast cancer (item b). Fig. 2 demonstrates that breast cancer patients (F2 to H2) tend to have higher fluorescence emission than healthy women (A1 to E3), corroborating the findings in Fig. 1.

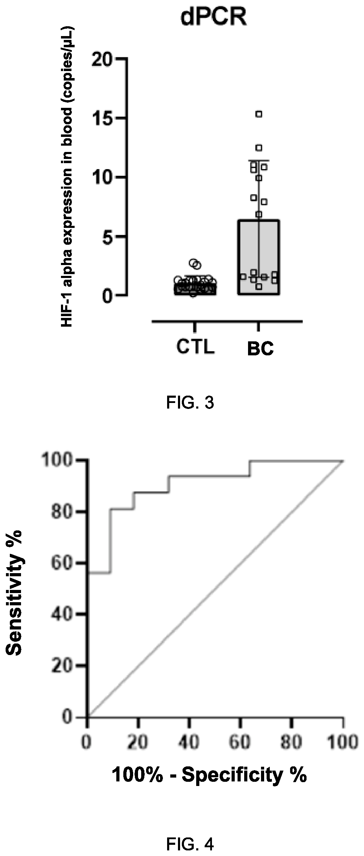

Fig. 3 refers to a graph of the number of copies/uL of HIF-1α in healthy women (CTL) and women with breast cancer (BC). It is noted that healthy women showed a mean of 1.18 (±0.66) copies/uL of HIF-1α transcripts, while women with breast cancer showed a mean of 6.46 copies/uL (±4.94) (p<0.0001).

With these data, an ROC curve was generated to evaluate the sensitivity and specificity of *HIF-1α* assessed by dPCR (Fig. 4). The area under the curve shown by *HIF-1α* was 0.9063 (95% CI = 0.8083 - 1.00; p<0.0001), with a cutoff value of 1,205 transcript copies/uL, with a sensitivity of 93.75% (95% CI = 71.67% to 99.68%) and a specificity of 68.18% (95% CI = 47.32% to 83.64%). These values generated by the ROC curve confirm the *HIF-1α* diagnostic value and its potential for use in liquid biopsy.

The promising results motivated the inventors to expand the study, including a larger number of patients and the quantitative assessment of several additional markers. The following examples show the results of the analyses.

### Example 2 - In vitro process for cancer diagnosis: quantification of HIF-1α in liquid biopsy using dPCR (study expansion)

### Study participants

The study included 270 women who were on the waiting list for mammography (real life study), comprising 235 women known to be cancer-free and 35 women with breast cancer already diagnosed and confirmed by histopathological test, all over 35 years of age. Each participant underwent an interview and provided 3 peripheral blood samples, namely: a) a collection tube with EDTA for complete blood count; b) a PaxGene collection tube (for gene expression study); and c) a collection tube for biochemical analysis. Next, the samples were stored and processed.

### RNA extraction from peripheral blood

For the total RNA extraction, peripheral blood samples were collected in PAXgene Blood RNA Tubes (PreAnalytiX, cat no. 762165), containing RNA stabilizers, were used. The extraction was performed using the PAXgene Blood RNA Kit (Qiagen cat. no. 762164), following the manufacturer's instructions. Subsequently, the total extracted RNA was quantified using a Qubit 4 fluorometer (ThermoFisher Scientific, cat no. Q33238), using the Qubit RNA BR Assay Kit (ThermoFisher Scientific, cat no. Q10211).

### cDNA synthesis

The cDNA was synthesized from 100 ng of total RNA using the QuantiTect Reverse Transcription kit (Qiagen, cat no. 205311), according to the manufacturer's protocol. Next, the synthesized cDNA was diluted 50X for amplification reactions.

The synthesized cDNA can also be diluted 25X for amplification reactions or not be diluted at all. Preferably, the synthesized cDNA is diluted 25X or 50X to avoid fluorescence saturation and allow for adequate visualization of the positive partitions.

### Gene expression analysis

To enable comparison of results using different techniques, the expression of the *HIF-1α* gene was evaluated by qPCR and dPCR. In qPCR reactions, HIF1-α expression was normalized by the expression values of the reference gene RPL13a. Other reference genes can also be used, such as glucuronidase beta (GUSB), transferrin receptor (TRFC), and β-actin. Specific primers for the genes of interest were designed using Primer3 Input 0.4 software, available at http://frodo.wi.mit.edu/primer3/. Subsequently, these designed primer sequences were checked for specificity using the Primer-BLAST program, available at http://www.ncbi.nlm.nih.gov/tools/primer-blast. The sequence of designed primers and the characteristics of their amplicons are described in Table 2.

**Table 2 - Characteristics of specific primers and probes and their amplicons.**

| Gene | SEQUENCE (5'-3') | AMPLICON (bp) |
|---|---|---|
| | F - SEQ ID NO:1 | |
| HIF-1α | R - SEQ ID NO:2 | 227 |
| | P - SEQ ID NO:3 | |
| | F - SEQ ID NO:4 | |
| RPL13a | R - SEQ ID NO:5 | 126 |
| | P - SEQ ID NO:6 | |
| | F - SEQ ID NO:7 | |
| GUSB | R - SEQ ID NO:8 | 63 |
| | P - SEQ ID NO:9 | |
| | F - SEQ ID NO:10 | |
| TRFC | R - SEQ ID NO:11 | 80 |
| | P - SEQ ID NO:12 | |
| | F - SEQ ID NO:13 | |
| B-actin | R - SEQ ID NO:14 | 83 |
| | P - SEQ ID NO:15 | |

The real-time PCR amplification reaction was performed in an Applied Biosystems 7500 Real Time PCR Systems thermocycler (Applied Biosystems, Foster City, CA, USA) in a final volume of 15 µL containing: 1X SYBR Green mix (Quantitec SYBR Green PCR kit, QIAGEN cat. no. 204054), 15 pmol of each specific primer and 1.5 µL cDNA. The thermocyclic parameters consisted of an initial hot start at 95°C for 10 minutes, followed by 40 repetitions of 95°C for 15 seconds and 60°C for 25 seconds. The arbitrary value of the expression was determined by formula (I): ${2}^{∧} \left(-ΔCq\right)$

The digital PCR (qPCR) amplification reaction was performed in a QIAcuity One Digital PCR System thermocycler (Qiagen cat no. 911021) using plates with 26,000 partitions per sample (Qiagen QlAcuity Nanoplate 26k 24-well, cat no. 250001) and plates with 8,500 partitions (QlAcuity Nanoplate 8.5k 24-*well,* cat no. 250011), in a final volume of 40 µL (plates with 26,000 partitions) or 12 µL (8.5K plates), containing: 1X EvaGreen (QIAcuity^{®} EG PCR Kit, Qiagen cat. no. 250112) or 1x Probe PCR Kit (QlAcuity Probe PCR kit, Qiagen cat. nos. 250102, 250101, 250103), 15 pmol of each specific primer (1.1X primer/probe set for HIF1a, 0.4X primer/probe set for RPL13a or 1X primer/probe set for HIF1a, 1X primer/probe set for TRFC or 1X primer/probe set for HIF1a, 1X primer/probe set for GUSB) and 2.0 µL cDNA. The thermocyclic parameters consist of an initial hot start step at 95°C for 2 minutes, followed by 40 repetitions of 95°C for 15 seconds, 60°C for 30 seconds, and 72°C for 15 seconds, ending with a 5-minute cooling step at 40°C. Fluorescence was captured by an exposure of 200 ms, with a gain value of 2.

### Statistical analysis

Initially, a descriptive analysis was performed in which the defined variables were expressed in absolute and relative frequencies, and the quantitative variables were summarized in means, standard deviations, minimum and maximum values. The Student's t-test was used to compare the mean differences between the groups of patients with and without breast cancer relative to the blood count and HIF-1α variables in the two collection periods and between the results obtained by qPCR and dPCR. The Pearson's correlation test was used to assess the correlation between HIF-1α biomarker expressions and blood count values in each sample collection. Analysis of variance (ANOVA) was performed to compare the differences in mean blood count and HIF-1α levels between liquid biopsy samples collected from each patient over time. A linear regression test was performed to evaluate the relationship between HIF-1α biomarker expression values and blood count values at each sample collection, taking into account the presence or absence of breast cancer. The statistical calculations were performed using NCSS software.

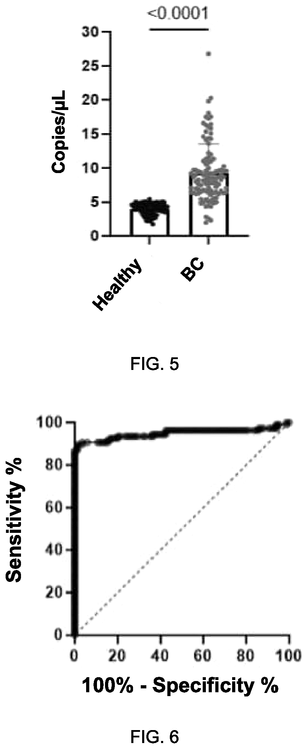

Fig. 5 refers to a graph of the number of copies/µL of HIF-1α in healthy women and women with breast cancer (BC). It is noted that healthy women showed a mean of 4.015 (±0.83) copies/uL of HIF-1α transcripts, while women with breast cancer showed a mean of 9.292 copies/uL (±4.240) (p<0.0001). The information in Fig. 5 is detailed in Table 3 below:

**Table 3**

| | Healthy | BC |
|---|---|---|
| Number of values | 85 | 107 |
| | | |
| Minimum | 1,770 | 2,001 |
| 25th percentile | 3,505 | 6,476 |
| Median | 4,150 | 8,489 |
| 75th percentile | 4,705 | 10.85 |
| Maximum | 5,430 | 26.79 |
| | | |
| Mean | 4,015 | 9,292 |
| Standard deviation | 0.8380 | 4,240 |

With this new dataset, a new ROC curve was generated to assess the sensitivity and specificity of *HIF-1α* by dPCR. Fig. 6 shows the ROC curve analysis for evaluating the accuracy of the absolute determination of the number of copies of *HIF-1α* transcripts normalized by the number of copies of the *TRFC* reference gene (calculation: HIF-1α / *TRFC*) in peripheral blood as a diagnostic breast cancer marker in liquid biopsy. The area under the curve shown by *HIF-1α* was 0.948 (95% CI = 0.9122 - 0.9844; p<0.0001), with a cutoff value for high risk >5,010 transcript copies/uL, with a sensitivity of 90.65% (95% CI = 83.65% to 94.84%) and a specificity of 96.47% (95% CI = 90.13% to 99.04%). This information from the ROC curve is detailed in Tables 4 and 5 below:

**Table 4**

| Area under the ROC curve | |
|---|---|
| Area | 0.9483 |
| Standard error | 0.01843 |
| 95% confidence interval | 0.9122 to 0.9844 |
| p-value | <0.0001 |

| Data | |
|---|---|
| Controls (healthy subjects) | 85 |
| Patients (BC) | 107 |
| Missing controls | 69 |
| Absent patients | 6 |

**Table 5**

| Cut-off Value | Sensitivity % | 95% Confidence Interval | Specificity % | 95% Confidence Interval | likelihood ratio |
|---|---|---|---|---|---|
| >4.785 | 93.46 | 87.11% to 96.80% | 78.82 | 68.99% to 86.16% | 4.431 |
| >4.812 | 93.46 | 87.11% to 96.80% | 80 | 70.28% to 87.12% | 4.676 |
| >4.817 | 92.52 | 85.94% to 96.16% | 80 | 70.28% to 87.12% | 4.626 |
| >4.845 | 92.52 | 85.94% to 96.16% | 81.18 | 71.59% to 88.07% | 4.915 |
| >4.88 | 92.52 | 85.94% to 96.16% | 82.35 | 72.90% to 89.00% | 5.243 |
| >4.891 | 92.52 | 85.94% to 96.16% | 83.53 | 74.23% to 89.93% | 5.617 |
| >4.911 | 91.59 | 84.78% to 95.51% | 83.53 | 74.23% to 89.93% | 5.561 |
| >4.932 | 91.59 | 84.78% to 95.51% | 84.71 | 75.57% to 90.84% | 5.988 |
| >4.942 | 90.65 | 83.65% to 94.84% | 84.71 | 75.57% to 90.84% | 5.927 |
| >4.955 | 90.65 | 83.65% to 94.84% | 85.88 | 76.93% to 91.74% | 6.421 |
| >4.965 | 90.65 | 83.65% to 94.84% | 87.06 | 78.30% to 92.62% | 7.005 |
| >4.975 | 90.65 | 83.65% to 94.84% | 88.74 | 79.68% to 93.48% | 7.706 |
| >4.985 | 90.65 | 83.65% to 94.84% | 89.41 | 81.09% to 94.33% | 8.562 |
| >4.995 | 90.65 | 83.65% to 94.84% | 94.12 | 86.96% to 97.46% | 15.41 |
| **>5.010** | **90.65** | **83.65% to 94.84%** | **96.47** | **90.13% to 99.04%** | **25.69** |
| >5.045 | 89.72 | 82.52% to 94.16% | 96.47 | 90.13% to 99.04% | 25.42 |
| >5.082 | 89.72 | 82.52% to 94.16% | 98.82 | 93.63% to 99.94% | 76.26 |
| >5.141 | 88.79 | 81.41% to 93.47% | 98.82 | 93.63% to 99.94% | 75.47 |
| >5.282 | 87.85 | 80.32% to 92.76% | 98.82 | 93.63% to 99.94% | 74.67 |
| >5.404 | 86.92 | 79.23% to 92.04% | 98.82 | 93.63% to 99.94% | 73.88 |
| >5.433 | 86.92 | 79.23% to 92.04% | 100 | 95.68% to 100.00% | |
| >5.472 | 85.98 | 78.15% to 91.32% | 100 | 95.68% to 100.00% | |
| >5.567 | 85.05 | 77.08% to 90.58% | 100 | 95.68% to 100.00% | |
| >5.859 | 84.11 | 76.02% to 89.84% | 100 | 95.68% to 100.00% | |
| >6.159 | 83.18 | 74.97% to 89.09% | 100 | 95.68% to 100.00% | |
| >6.233 | 82.24 | 73.92% to 88.33% | 100 | 95.68% to 100.00% | |
| >6.261 | 81.31 | 72.89% to 87.56% | 100 | 95.68% to 100.00% | |

The data shown herein was obtained with a statistically relevant sample size and confirm the applicability of *HIF-1α* as a diagnostic breast cancer marker in liquid biopsy.

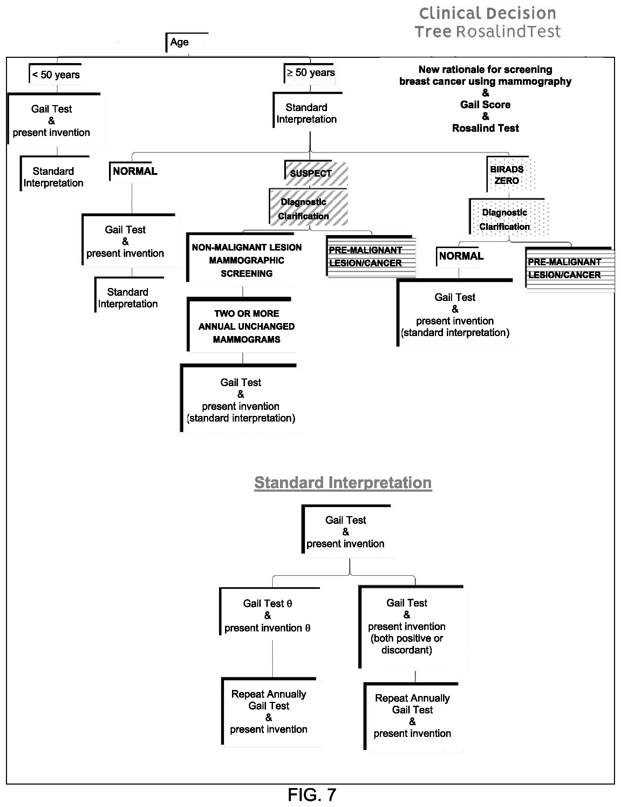

Fig. 7 refers to a decision tree that incorporates all possible applications of the present invention in the various clinical scenarios of the patients. As mentioned earlier, breast density makes it difficult to accurately analyze mammography results in women under 50 years of age; therefore, the present invention represents a solution for monitoring and screening breast cancer in these younger women. The present invention can be combined with the Gail score, which assesses risk factors such as age, age at menarche, age at first pregnancy, family history of breast cancer, number of breast biopsies performed with or without atypical hyperplasia, and ethnicity. In cases where both tests yield negative results for the presence of breast cancer, annual repeat testing may be recommended. In cases where both tests provide positive or discordant results regarding the presence of breast cancer, mammography screening may be recommended.

Furthermore, the present invention may be an alternative method for diagnosing and screening breast cancer in women over 50 years of age and complement mammography analysis when results are interpreted as normal, suspicious, or inconclusive.

### Example 3 - Evaluation of the markers MCT1, MCT4 and CD147

To verify the use of a marker panel in the screening, diagnosis and/or prognosis of breast cancer, in this example a study was carried out with the markers *MCT1, MCT4,* and *CD147.*

This study was conducted according to the protocol available in the article: Evaluation of MCT1, MCT4 and CD147 Genes in Peripheral Blood Cells of Breast Cancer Patients and Their Potential Use as Diagnostic and Prognostic Markers. (doi: 10.3390/ijms18040170).

For each patient, peripheral blood samples were analyzed at the time of diagnosis (collection 1), and 3 and 6 months after the start of chemotherapy treatment (collections 2 and 3, respectively), as well as biopsy samples from the original tumor. As a control, peripheral blood samples were used from healthy women (donors), over 18 years of age, with no history of breast cancer (confirmed by annual routine screening for breast cancer) or other diseases, diabetes, kidney and/or cardiovascular diseases.

The expression of the target gene was determined by formula (I), where ΔCq corresponds to the difference between the Cq values of the target gene and the endogenous (reference) gene. ${2}^{∧} - \left(ΔCq\right)$

**Table 6 - MCT1 data, progression and staging**

| | | | **MCT1 (2^-(DCq)** | | | |
|---|---|---|---|---|---|---|
| **patient** | **Progression*** | **Staging^{#}** | **MCT1 (coll. 1)** | **MCT1 (coll. 2)** | **MCT1 (coll. 3)** | **TUMOR MCT1** |
| 1 | 0 | 1 | 0.014 | 0.002 | 0.049 | 0.00 |
| 7 | 1 | 2 | 0.073 | 0.028 | 0.013 | 0.00 |
| 8 | 0 | 2 | 0.107 | 0.081 | 0.283 | 0.00 |
| 9 | 0 | 1 | 0.009 | 0.006 | 0.010 | 0.00 |
| 11 | 0 | 2 | 1.000 | 0.014 | 0.006 | 0.00 |
| 12 | 0 | 2 | 0.007 | 0.040 | 0.104 | |
| 13 | 0 | 3 | 0.082 | | 0.420 | 1.12 |
| 15 | 0 | 2 | 0.012 | 0.009 | 0.047 | 0.00 |
| 16 | 0 | 2 | 0.011 | 0.009 | 0.024 | 0.00 |
| 17 | 1 | 2 | 0.006 | 0.013 | 0.061 | |
| 18 | 0 | 1 | 0.014 | 0.008 | 0.002 | 12.09 |
| 21 | 0 | 2 | 0.004 | 0.020 | 0.010 | 23.92 |
| 22 | 0 | 2 | 0.017 | 0.054 | 0.004 | 14.71 |
| 24 | 0 | 1 | 0.020 | 0.030 | 0.293 | |
| 25 | 0 | 3 | 0.020 | 0.088 | 0.004 | 2.20 |
| 26 | 0 | 2 | 0.016 | 0.058 | 0.168 | 1.00 |
| 27 | 0 | 3 | 0.052 | 0.010 | 0.055 | 0.53 |
| 28 | 1 | 3 | 0.041 | 0.030 | 0.435 | 0.00 |
| 29 | 0 | 1 | 0.008 | 0.192 | 0.030 | |
| 30 | 0 | 1 | 0.001 | 0.000 | 0.000 | 4.90 |
| 32 | 0 | 1 | 0.000 | 0.000 | 0.000 | 74.09 |
| 33 | 0 | 3 | 0.000 | 0.000 | 0.000 | 0.00 |
| 35 | 0 | 1 | 0.000 | 0.000 | 0.000 | 0.00 |
| 36 | 0 | 3 | 0.000 | 0.000 | 0.000 | 0.00 |
| 37 | 0 | 3 | 0.000 | 0.000 | 0.000 | 0.00 |
| 38 | 0 | 2 | 0.000 | 0.000 | 0.002 | 0.00 |
| 39 | 0 | 3 | 0.000 | 0.000 | 0.001 | 15.88 |
| 40 | 0 | 3 | 0.000 | 0.000 | 0.000 | |
| 41 | 0 | 3 | 0.056 | 0.013 | 0.016 | 37.30 |
| 42 | 0 | 2 | 0.039 | | 0.149 | 591.55 |
| 43 | 0 | 1 | 0.050 | 0.035 | 0.159 | 0.00 |
| 45 | 0 | 2 | 0.009 | 0.026 | 0.043 | 0.00 |
| 47 | 0 | 3 | 0.033 | 0.005 | 0.003 | 0.00 |
| 48 | 0 | 3 | 0.003 | 0.016 | 0.010 | 6.52 |
| 49 | 0 | 2 | 0.032 | 0.009 | 0.013 | 0.00 |
| 50 | 0 | 3 | 0.038 | 0.005 | 0.044 | 5.67 |
| 52 | 0 | 1 | 0.017 | 0.009 | 0.607 | 0.00 |
| 54 | 0 | 2 | 0.027 | 0.011 | 0.323 | 1.09 |
| 56 | 0 | 2 | 0.020 | 0.034 | 0.008 | 8.63 |
| 57 | 0 | 1 | 1.000 | 0.049 | 0.007 | |
| 58 | 0 | 3 | 0.027 | 0.022 | 0.104 | 0.00 |
| 60 | 0 | 3 | 0.052 | 0.025 | 0.331 | 63.62 |
| 61 | 1 | 3 | 0.048 | 0.036 | 0.015 | 5.68 |
| 62 | 0 | 2 | 0.026 | 0.066 | 0.007 | 17.85 |
| 64 | 0 | 3 | 0.019 | 0.144 | 0.032 | 7.68 |
| 65 | 0 | 1 | 1.000 | 0.039 | 0.481 | 0.00 |
| 67 | 0 | 2 | 0.020 | 0.010 | 0.009 | 0.44 |
| 68 | 0 | 2 | 0.007 | 0.005 | 0.007 | 4.47 |
| 71 | 0 | 2 | 0.004 | 0.021 | 0.543 | 7.86 |
| 72 | 0 | 3 | 0.014 | 0.006 | 0.039 | 6.06 |
| 74 | 0 | 2 | 0.070 | 0.009 | 0.005 | 0.00 |
| 76 | 1 | 2 | 0.021 | 0.503 | 0.309 | 0.05 |
| 78 | | | 0.018 | 0.007 | 0.004 | 7.37 |
| 79 | 0 | 2 | 0.019 | 0.012 | 0.077 | 8.96 |
| 80 | 0 | 2 | 0.008 | 0.504 | 0.055 | 0.48 |
| 81 | 0 | 2 | 0.001 | 0.004 | 0.003 | 5.63 |
| 82 | 0 | 2 | 0.012 | 0.008 | 0.004 | 1.81 |
| 83 | 0 | 2 | 0.020 | 0.002 | 0.008 | 70.78 |
| 84 | 0 | 2 | 0.022 | 0.007 | 0.015 | 0.01 |
| 85 | 1 | 2 | 0.004 | 0.017 | 0.010 | 1.53 |
| 86 | 1 | 3 | 0.018 | 0.001 | 0.001 | 0.02 |
| 87 | 0 | 3 | 0.003 | 0.019 | 0.069 | 0.00 |
| 88 | 0 | 2 | 0.024 | 0.064 | 0.117 | |
| 89 | 1 | 3 | 0.003 | 0.003 | 0.005 | 0.51 |
| 90 | 0 | 3 | 0.005 | 0.001 | 1.000 | 2.39 |
| 93 | 0 | 1 | 0.001 | 0.001 | 0.033 | 0.48 |
| 94 | 0 | 1 | 0.002 | 0.006 | 0.028 | |
| 95 | 0 | 1 | 0.001 | 1.000 | 0.004 | |
| 96 | 0 | 2 | 0.009 | 0.006 | 1.000 | |
| 97 | 0 | 1 | 0.003 | 0.001 | 0.012 | |
| 99 | 0 | 1 | 0.019 | 0.002 | 0.002 | 68.78 |
| 101 | 1 | 3 | 0.370 | 0.001 | 0.001 | 144.63 |
| 102 | 0 | 3 | 0.002 | 0.001 | 0.012 | 62.64 |
| 106 | 0 | | 0.001 | 0.002 | 0.000 | |
| 107 | 0 | 2 | 0.001 | 0.001 | 0.000 | |
| 109 | 0 | 2 | 0.001 | 0.004 | 0.005 | |
| 110 | 0 | 2 | 0.002 | 0.001 | 0.003 | 125.03 |
| 111 | 0 | 1 | 0.001 | 0.001 | 0.002 | 32.27 |
| 112 | 0 | 2 | 0.001 | 0.001 | 0.001 | 55.59 |
| 114 | 0 | 2 | 0.001 | 0.004 | 0.002 | 30.57 |
| 116 | 0 | 2 | 0.001 | 0.001 | 0.001 | 23.29 |
| 117 | 0 | 2 | 0.001 | 0.001 | 0.000 | |
| 118 | | | 0.000 | 0.001 | 0.001 | |
| 119 | 0 | 2 | 0.001 | 0.000 | 0.002 | |
| 120 | 0 | 2 | 0.203 | 0.000 | 0.000 | 0.61 |
| 121 | 0 | 1 | 0.000 | 0.000 | 0.000 | |
| 122 | 0 | 2 | 0.000 | 0.000 | 0.001 | 0.09 |
| 127 | 0 | 2 | 0.000 | 0.001 | 0.000 | |
| 128 | 0 | 3 | 0.001 | 0.000 | 0.000 | |
| 129 | 0 | 1 | 0.000 | 0.000 | 0.001 | |
| 130 | 0 | 2 | 0.000 | 0.003 | 0.004 | |
| 131 | 0 | 2 | 0.000 | 0.000 | 0.001 | |
| 132 | 0 | 1 | 1.000 | 0.000 | 0.000 | |
| 136 | 0 | 2 | 0.000 | 0.000 | 0.000 | |
| 137 | 0 | 2 | 0.000 | 0.000 | 0.004 | |
| 140 | 0 | 2 | 0.000 | 0.000 | 0.000 | |
| 141 | 0 | 2 | 0.000 | 0.001 | 0.000 | |
| 142 | 0 | 3 | 0.000 | 0.000 | 0.001 | |
| 144 | 0 | | 0.000 | 0.000 | 0.000 | |
| 145 | 0 | 2 | 0.000 | 0.000 | 0.004 | |
| 149 | 0 | 2 | 0.002 | 0.001 | 0.001 | |
| 150 | 0 | 2 | 0.005 | 0.000 | 0.006 | |
| 151 | 0 | 1 | 0.001 | 0.007 | 0.004 | |
| 152 | 0 | 3 | 0.013 | 0.001 | 0.001 | |
| 154 | 0 | 3 | 0.003 | 0.001 | 0.623 | |
| 157 | 0 | | 0.001 | 0.001 | 0.014 | |
| 158 | 0 | 2 | 0.008 | 0.003 | 0.001 | |
| 159 | 0 | 1 | 0.005 | 0.016 | 0.010 | |
| 160 | 0 | 1 | 0.018 | 0.008 | 0.041 | |
| 163 | 0 | 2 | 0.000 | 0.000 | 0.000 | |
| 164 | 0 | 1 | 0.000 | 0.000 | 0.000 | |
| 167 | 0 | 3 | 0.000 | 0.000 | 0.000 | |
| 168 | 0 | 1 | 0.000 | 0.000 | 0.001 | |
| 169 | 0 | | 0.000 | 0.000 | 0.001 | |
| 170 | 0 | 1 | 0.000 | 0.000 | 0.000 | |
| 171 | 1 | 2 | 0.000 | 0.000 | 0.000 | |
| 173 | 0 | 2 | 0.000 | 0.000 | 0.000 | |
| 176 | 0 | 2 | 0.000 | 0.002 | 0.001 | 0.00 |
| 177 | 0 | 2 | 0.004 | 0.002 | 0.003 | |
| 178 | 0 | 2 | 0.008 | 0.008 | 0.097 | |
| 179 | 0 | 2 | 0.006 | 0.003 | 0.019 | |
| 184 | 0 | 2 | 0.001 | 0.001 | 0.005 | |
| 186 | 0 | 0 | 0.000 | 0.024 | 0.002 | |
| 187 | 0 | 1 | 0.001 | 0.009 | 0.001 | |
| 191 | 0 | 3 | 0.002 | 0.001 | 0.020 | |

| | | | | | | |
|---|---|---|---|---|---|---|
| *Progression 0: negative; Progression 1: positive. ^{#}Staging: in ascending order refers to the progression of breast cancer. | | | | | | |

**Table 7 - MCT4 data, progression and staging**

| | | | **MCT4 (2^-(DCq)** | | | |
|---|---|---|---|---|---|---|
| **patient** | **Progression*** | **Staging^{#}** | **MCT4 (coll. 1)** | **MCT4 (coll. 2)** | **MCT4 (coll. 3)** | **MCT4 TUMOR** |
| 1 | 0 | 1 | 0.04 | 0.05 | 0.087 | 0.000 |
| 7 | 1 | 2 | 0.01 | 0.02 | 0.019 | |
| 8 | 0 | 2 | 0.39 | 0.02 | 0.025 | 0.000 |
| 9 | 0 | 1 | 0.11 | 0.01 | 0.044 | 0.000 |
| 11 | 0 | 2 | | 0.25 | 0.006 | |
| 12 | 0 | 2 | 0.02 | 0.01 | 0.004 | |
| 13 | 0 | 3 | 0.03 | | 0.399 | 6.684 |
| 15 | 0 | 2 | 0.01 | 0.01 | 0.003 | |
| 16 | 0 | 2 | 0.01 | 0.04 | 0.009 | 0.000 |
| 17 | 1 | 2 | 0.01 | 0.02 | 0.015 | 0.000 |
| 18 | 0 | 1 | 0.00 | 0.00 | 0.008 | 10.797 |
| 21 | 0 | 2 | 0.01 | 0.00 | 0.004 | 8.011 |
| 22 | 0 | 2 | 0.01 | 0.01 | 0.003 | |
| 24 | 0 | 1 | 0.01 | 0.01 | 0.016 | |
| 25 | 0 | 3 | 0.01 | 0.00 | 0.001 | 5.647 |
| 26 | 0 | 2 | 0.01 | 0.01 | 0.009 | |
| 27 | 0 | 3 | 0.01 | 0.01 | 0.005 | 0.053 |
| 28 | 1 | 3 | 0.01 | 0.00 | 0.457 | 0.000 |
| 29 | 0 | 1 | 0.00 | 0.04 | 0.007 | |
| 30 | 0 | 1 | 0.00 | 0.00 | 0.000 | 44.185 |
| 32 | 0 | 1 | 0.00 | 0.00 | 0.000 | 98.111 |
| 33 | 0 | 3 | 0.00 | 0.00 | 0.000 | 0.000 |
| 35 | 0 | 1 | 0.00 | 0.00 | 0.000 | 0.000 |
| 36 | 0 | 3 | 0.00 | 0.00 | 0.000 | 0.000 |
| 37 | 0 | 3 | 0.00 | 0.00 | 0.000 | 0.000 |
| 38 | 0 | 2 | 0.00 | 0.00 | 0.000 | 0.000 |
| 39 | 0 | 3 | 0.00 | 0.00 | 0.000 | 32.842 |
| 40 | 0 | 3 | 0.00 | 0.00 | 0.003 | |
| 41 | 0 | 3 | 0.01 | 0.01 | 0.006 | 27.249 |
| 42 | 0 | 2 | 0.01 | 0.00 | 0.003 | 40.794 |
| 43 | 0 | 1 | 0.01 | 0.00 | 0.003 | |
| 45 | 0 | 2 | 0.00 | 0.00 | 0.005 | 0.000 |
| 47 | 0 | 3 | 0.01 | 0.00 | 0.001 | |
| 48 | 0 | 3 | 0.00 | 0.00 | 0.009 | |
| 49 | 0 | 2 | 0.01 | 0.01 | 0.520 | 0.005 |
| 50 | 0 | 3 | 0.01 | 0.25 | 0.121 | 2.237 |
| 52 | 0 | 1 | 0.01 | 0.52 | 0.146 | 0.000 |
| 54 | 0 | 2 | 1.13 | 0.29 | 4.119 | 112.583 |
| 56 | 0 | 2 | 0.20 | 0.49 | 1.730 | 65.083 |
| 57 | 0 | 1 | 0.29 | 0.04 | 0.196 | |
| 58 | 0 | 3 | 0.77 | 0.25 | 0.238 | |
| 60 | 0 | 3 | 0.81 | 0.38 | 0.475 | 4.537 |
| 61 | 1 | 3 | 0.62 | 0.17 | 0.628 | 1.901 |
| 62 | 0 | 2 | 0.85 | 0.55 | 0.001 | 5.825 |
| 64 | 0 | 3 | 0.65 | 0.00 | 0.001 | 175.384 |
| 65 | 0 | 1 | 0.77 | 0.00 | 0.007 | 0.000 |
| 67 | 0 | 2 | 0.00 | 0.00 | 0.003 | 1.522 |
| 68 | 0 | 2 | 0.01 | 0.00 | 0.003 | 1.085 |
| 71 | 0 | 2 | 0.01 | 0.01 | 0.008 | 0.973 |
| 72 | 0 | 3 | 0.01 | 0.01 | 0.007 | 2.183 |
| 74 | 0 | 2 | 0.00 | 0.01 | 0.002 | 0.014 |
| 76 | 1 | 2 | 0.01 | 0.01 | 0.001 | 0.023 |
| 78 | | | 0.00 | 0.01 | 0.860 | 25.303 |
| 79 | 0 | 2 | 0.01 | 0.10 | 0.069 | 3.148 |
| 80 | 0 | 2 | 0.01 | 0.36 | 0.176 | 31.260 |
| 81 | 0 | 2 | 0.05 | 0.13 | 0.167 | 4.813 |
| 82 | 0 | 2 | 0.15 | 0.14 | 0.112 | 51.299 |
| 83 | 0 | 2 | 0.13 | 1.38 | 0.015 | 93.130 |
| 84 | 0 | 2 | 0.15 | 0.03 | 0.044 | 0.045 |
| 85 | 1 | 2 | 0.16 | 1.38 | 0.112 | 20.826 |
| 86 | 1 | 3 | 0.27 | 0.03 | 0.015 | 0.015 |
| 87 | 0 | 3 | 0.43 | 0.81 | 0.044 | |
| 88 | 0 | 2 | 0.16 | 0.13 | 0.213 | |
| 89 | 1 | 3 | 0.02 | 0.04 | 0.058 | 0.509 |
| 90 | 0 | 3 | 0.03 | 0.02 | 1.000 | 1.094 |
| 93 | 0 | 1 | 0.01 | 0.02 | 0.029 | 58.188 |
| 94 | 0 | 1 | 0.02 | 0.05 | 0.012 | |
| 95 | 0 | 1 | 0.08 | 0.03 | 0.024 | |
| 96 | 0 | 2 | 0.06 | 0.01 | 1.000 | |
| 97 | 0 | 1 | 0.04 | 0.00 | 0.055 | |
| 99 | 0 | 1 | 0.09 | 0.04 | 0.097 | 4.180 |
| 101 | 1 | 3 | 0.31 | 0.01 | 0.003 | |
| 102 | 0 | 3 | 0.01 | 0.01 | 0.027 | |
| 106 | 0 | | 0.00 | 0.00 | 0.000 | |
| 107 | 0 | 2 | 0.00 | 0.00 | 0.001 | |
| 109 | 0 | 2 | 0.00 | 0.00 | 0.001 | |
| 110 | 0 | 2 | 0.00 | 0.00 | 0.000 | |
| 111 | 0 | 1 | 0.00 | 0.00 | 0.001 | |
| 112 | 0 | 2 | 0.00 | 0.00 | 0.001 | |
| 114 | 0 | 2 | 0.00 | 0.01 | 0.001 | |
| 116 | 0 | 2 | 0.00 | 0.00 | 0.001 | |
| 117 | 0 | 2 | 0.00 | 0.00 | 0.001 | |
| 118 | | | 0.00 | 0.00 | 0.000 | |
| 119 | 0 | 2 | 0.00 | 0.00 | 0.000 | |
| 120 | 0 | 2 | 0.04 | 0.00 | 0.000 | |
| 121 | 0 | 1 | 0.00 | 0.00 | 0.000 | |
| 122 | 0 | 2 | 0.00 | 0.00 | 0.000 | |
| 127 | 0 | 2 | 0.00 | 0.00 | 0.000 | |
| 128 | 0 | 3 | 0.00 | 0.00 | 0.000 | |
| 129 | 0 | 1 | 0.00 | 0.00 | 0.000 | |
| 130 | 0 | 2 | 0.00 | 0.00 | 0.000 | |
| 131 | 0 | 2 | 0.00 | 0.00 | 0.000 | |
| 132 | 0 | 1 | 0.00 | 0.00 | 0.000 | |
| 136 | 0 | 2 | 0.00 | 0.00 | 0.000 | |
| 137 | 0 | 2 | 0.00 | 0.00 | 0.001 | |
| 140 | 0 | 2 | 0.00 | 0.00 | 0.000 | |
| 141 | 0 | 2 | 0.00 | 0.00 | 0.000 | |
| 142 | 0 | 3 | 0.00 | 0.00 | 0.000 | |
| 144 | 0 | | 0.00 | 0.00 | 0.000 | |
| 145 | 0 | 2 | 0.00 | 0.00 | 0.001 | |
| 149 | 0 | 2 | 0.02 | 0.00 | 0.006 | |
| 150 | 0 | 2 | 0.01 | 0.00 | 0.016 | |
| 151 | 0 | 1 | 0.02 | 0.02 | 0.013 | |
| 152 | 0 | 3 | 0.03 | 0.00 | 0.001 | |
| 154 | 0 | 3 | 0.01 | 0.01 | 0.220 | |
| 157 | 0 | | 0.00 | 0.00 | 0.021 | |
| 158 | 0 | 2 | 0.02 | 0.00 | 0.003 | |
| 159 | 0 | 1 | 0.03 | 0.01 | 0.060 | |
| 160 | 0 | 1 | 0.02 | 0.02 | 0.026 | |
| 163 | 0 | 2 | 0.00 | 0.00 | 0.000 | |
| 164 | 0 | 1 | 0.00 | 0.00 | 0.000 | |
| 167 | 0 | 3 | 0.00 | 0.00 | 0.000 | |
| 168 | 0 | 1 | 0.00 | 0.00 | 0.000 | |
| 169 | 0 | | 0.00 | 0.00 | 0.000 | |
| 170 | 0 | 1 | 0.00 | 0.00 | 0.000 | |
| 171 | 1 | 2 | 0.00 | 0.00 | 0.000 | |
| 173 | 0 | 2 | 0.00 | 0.00 | 1.000 | |
| 176 | 0 | 2 | 0.00 | 0.00 | 0.000 | |
| 177 | 0 | 2 | 0.00 | 0.00 | 0.002 | |
| 178 | 0 | 2 | 0.02 | 0.00 | 0.005 | |
| 179 | 0 | 2 | 0.01 | 0.00 | 0.006 | |
| 184 | 0 | 2 | 0.00 | 0.00 | 0.001 | |
| 186 | 0 | 0 | 0.00 | 0.01 | 0.005 | |
| 187 | 0 | 1 | 0.00 | 0.00 | 0.001 | |
| 191 | 0 | 3 | 0.01 | 0.00 | 0.002 | |

| | | | | | | |
|---|---|---|---|---|---|---|
| *Progression 0: negative; Progression 1: positive. ^{#}Staging: in ascending order refers to the progression of breast cancer. | | | | | | |

**Table 8 - CD147 data, progression and staging**

| | | | **CD147 (2^-(DCq)** | | | |
|---|---|---|---|---|---|---|
| **patient** | **progression*** | **staging^{#}** | **CD147 (coll. 1)** | **CD147 (coll. 2)** | **CD147 (coll. 3)** | **CD147 TUMOR** |
| 1 | 0 | 1 | 3.79 | 2.73 | 0.28 | 3.38785E-07 |
| 7 | 1 | 2 | 3.70 | 0.37 | 0.89 | 9.16166E-08 |
| 8 | 0 | 2 | 35.23 | 3.61 | 2.61 | 8.82501E-07 |
| 9 | 0 | 1 | 14.40 | 4.65 | 11.44 | |
| 11 | 0 | 2 | | 17.87 | 3.33 | 1.30962E-06 |
| 12 | 0 | 2 | 4.11 | 4.30 | 1.67 | |
| 13 | 0 | 3 | 5.03 | | 25.28 | 1514.034925 |
| 15 | 0 | 2 | 3.61 | 1.90 | 4.20 | 7.02476E-05 |
| 16 | 0 | 2 | 2.03 | 1.99 | 2.03 | 7.39767E-06 |
| 17 | 1 | 2 | 2.99 | 4.18 | 3.18 | 2.50869E-05 |
| 18 | 0 | 1 | 0.65 | 0.76 | 1.67 | |
| 21 | 0 | 2 | 0.77 | 0.40 | 0.47 | |
| 22 | 0 | 2 | 0.73 | 1.29 | 1.04 | |
| 24 | 0 | 1 | 1.01 | 0.93 | 0.97 | |
| 25 | 0 | 3 | 0.72 | 0.90 | 0.47 | |
| 26 | 0 | 2 | 1.10 | 0.92 | 0.46 | |
| 27 | 0 | 3 | 0.81 | 0.50 | 0.62 | 0.072390902 |
| 28 | 1 | 3 | 1.10 | 1.14 | 1.07 | 7.60973E-06 |
| 29 | 0 | 1 | 0.38 | 0.99 | 0.86 | |
| 30 | 0 | 1 | 0.41 | 0.22 | 0.19 | |
| 32 | 0 | 1 | 0.20 | 0.07 | 0.13 | |
| 33 | 0 | 3 | 0.09 | 0.11 | 0.15 | 2.17059E-05 |
| 35 | 0 | 1 | 0.17 | 0.09 | 0.17 | 3.1382E-05 |
| 36 | 0 | 3 | 0.15 | 0.15 | 0.14 | 3.69082E-05 |
| 37 | 0 | 3 | 0.10 | 0.07 | 0.08 | 5.25128E-06 |
| 38 | 0 | 2 | 0.31 | 0.25 | 0.14 | 8.67615E-06 |
| 39 | 0 | 3 | 0.16 | 0.19 | 0.15 | |
| 40 | 0 | 3 | 0.23 | 0.20 | 0.06 | |
| 41 | 0 | 3 | 26.19 | 29.85 | 23.00 | |
| 42 | 0 | 2 | 13.52 | 11.94 | 16.90 | |
| 43 | 0 | 1 | 24.26 | 20.09 | 8.14 | 1.15835E-05 |
| 45 | 0 | 2 | 14.80 | 10.02 | 13.26 | 2.17607E-06 |
| 47 | 0 | 3 | 13.84 | 4.99 | 6.60 | 0.001172418 |
| 48 | 0 | 3 | 23.38 | 25.74 | 17.41 | |
| 49 | 0 | 2 | 20.17 | 11.35 | 14.58 | 0.053734978 |
| 50 | 0 | 3 | 27.98 | 13.54 | 12.86 | 277.0360185 |
| 52 | 0 | 1 | 14.37 | 7.16 | 14.11 | 0.000102001 |
| 54 | 0 | 2 | 3.59 | 3.75 | 2.81 | |
| 56 | 0 | 2 | 5.18 | 12.36 | 3.10 | |
| 57 | 0 | 1 | 6.77 | 3.25 | 9.28 | |
| 58 | 0 | 3 | 6.61 | 2.68 | 22.38 | 0.000778871 |
| 60 | 0 | 3 | 15.62 | 3.68 | 11.74 | 0.415417961 |
| 61 | 1 | 3 | 7.26 | 5.22 | 4.32 | 247.336068 |
| 62 | 0 | 2 | 5.71 | 3.76 | 2.82 | |
| 64 | 0 | 3 | 5.04 | 5.83 | 11.39 | |
| 65 | 0 | 1 | 8.82 | 5.57 | 3.12 | 2.52721E-05 |
| 67 | 0 | 2 | 8.00 | 2.55 | 2.96 | |
| 68 | 0 | 2 | 4.05 | 6.03 | 4.57 | |
| 71 | 0 | 2 | 3.65 | 3.77 | 1.25 | |
| 72 | 0 | 3 | 10.00 | 5.42 | 9.31 | |
| 74 | 0 | 2 | 21.06 | 9.71 | 7.87 | 5.099463989 |
| 76 | 1 | 2 | 6.64 | 5.07 | 1.23 | 0.850408382 |
| 78 | | | 5.38 | 16.05 | 4.51 | |
| 79 | 0 | 2 | 6.38 | 4.66 | 6.11 | |
| 80 | 0 | 2 | 11.71 | 3.32 | 0.74 | |
| 81 | 0 | 2 | 1.41 | 1.49 | 1.89 | |
| 82 | 0 | 2 | 2.10 | 8.21 | 1.69 | |
| 83 | 0 | 2 | 1.55 | 1.25 | 3.54 | |
| 84 | 0 | 2 | 5.09 | 5.67 | 2.40 | 0.013140622 |
| 85 | 1 | 2 | 2.04 | 19.89 | 1.90 | 0.489037695 |
| 86 | 1 | 3 | 1.75 | 3.71 | 2.08 | 0.004227033 |
| 87 | 0 | 3 | 3.54 | 2.35 | 0.72 | 0.006177846 |
| 88 | 0 | 2 | 1.95 | 2.28 | 0.48 | |
| 89 | 1 | 3 | 1.92 | 2.18 | 2.94 | 26.20414856 |
| 90 | 0 | 3 | 1.79 | 0.71 | 1.00 | |
| 93 | 0 | 1 | 1.40 | 0.96 | 5.34 | |
| 94 | 0 | 1 | 1.34 | 2.81 | 0.07 | |
| 95 | 0 | 1 | 1.77 | 2.03 | 1.34 | 4.425798962 |
| 96 | 0 | 2 | 2.68 | 1.53 | 1.00 | |
| 97 | 0 | 1 | 2.87 | 1.36 | 5.14 | |
| 99 | 0 | 1 | 1.13 | 3.15 | 1.18 | |
| 101 | 1 | 3 | 16.75 | 1.43 | 0.68 | |
| 102 | 0 | 3 | 1.53 | 0.87 | 1.05 | |
| 106 | 0 | | 4.13 | 8.26 | 1.41 | |
| 107 | 0 | 2 | 3.75 | 4.35 | 2.20 | |
| 109 | 0 | 2 | 2.28 | 1.27 | 3.94 | |
| 110 | 0 | 2 | 4.24 | 7.71 | 1.14 | |
| 111 | 0 | 1 | 10.22 | 12.24 | 0.32 | |
| 112 | 0 | 2 | 3.70 | 5.15 | 0.00 | |
| 114 | 0 | 2 | 9.86 | 3.49 | 1.77 | |
| 116 | 0 | 2 | 10.01 | 1.36 | 27.22 | 1.195884137 |
| 117 | 0 | 2 | 8.18 | 6.11 | 1.35 | |
| 118 | | | 2.94 | 6.28 | 2.40 | |
| 119 | 0 | 2 | 2.93 | 1.23 | 0.68 | |
| 120 | 0 | 2 | | 0.36 | 0.54 | |
| 121 | 0 | 1 | 0.69 | 0.18 | 0.43 | |
| 122 | 0 | 2 | 0.72 | 1.18 | 0.36 | 0.11228386 |
| 127 | 0 | 2 | 1.29 | 1.19 | 0.17 | |
| 128 | 0 | 3 | 0.79 | 0.99 | 0.48 | |
| 129 | 0 | 1 | 1.11 | 0.46 | 0.93 | |
| 130 | 0 | 2 | 0.37 | 0.36 | 0.53 | |
| 131 | 0 | 2 | 2.25 | 1.01 | 0.49 | |
| 132 | 0 | 1 | 0.77 | 0.87 | 1.05 | |
| 136 | 0 | 2 | 1.20 | 1.84 | 2.41 | |
| 137 | 0 | 2 | 1.69 | 0.64 | 0.67 | |
| 140 | 0 | 2 | 1.55 | 0.69 | 0.95 | |
| 141 | 0 | 2 | 2.06 | 1.40 | 0.98 | |
| 142 | 0 | 3 | 1.98 | 1.25 | 1.28 | |
| 144 | 0 | | 1.20 | 1.27 | 1.48 | |
| 145 | 0 | 2 | 1.77 | 0.69 | 0.64 | |
| 149 | 0 | 2 | 0.02 | 0.02 | 0.02 | |
| 150 | 0 | 2 | 0.05 | 0.00 | 0.02 | |
| 151 | 0 | 1 | 0.03 | 0.05 | 0.00 | |
| 152 | 0 | 3 | 0.04 | 0.01 | 0.01 | |
| 154 | 0 | 3 | 0.00 | 0.01 | 0.22 | |
| 157 | 0 | | 0.02 | 0.02 | 0.02 | |
| 158 | 0 | 2 | 0.03 | 0.01 | 0.00 | |
| 159 | 0 | 1 | 0.03 | 0.01 | 0.32 | |
| 160 | 0 | 1 | 0.06 | 0.02 | 0.02 | |
| 163 | 0 | 2 | 0.05 | 0.01 | 0.04 | |
| 164 | 0 | 1 | 0.05 | 0.01 | 0.01 | |
| 167 | 0 | 3 | 0.04 | 0.22 | 0.02 | |
| 168 | 0 | 1 | 0.00 | 0.04 | 0.01 | |
| 169 | 0 | | 0.04 | 0.03 | 0.03 | |
| 170 | 0 | 1 | 0.07 | 0.04 | 0.12 | |
| 171 | 1 | 2 | 0.02 | 0.02 | 0.06 | |
| 173 | 0 | 2 | 0.07 | 0.03 | 0.04 | |
| 176 | 0 | 2 | 0.12 | 0.52 | 0.62 | |
| 177 | 0 | 2 | 0.39 | 0.19 | 0.31 | |
| 178 | 0 | 2 | 0.76 | 0.60 | 0.25 | |
| 179 | 0 | 2 | 1.15 | 0.25 | 1.32 | |
| 184 | 0 | 2 | 1.14 | 0.83 | 1.74 | |
| 186 | 0 | 0 | 0.17 | 2.35 | 0.57 | |
| 187 | 0 | 1 | 0.28 | 0.93 | 0.73 | |
| 191 | 0 | 3 | 1.19 | 0.58 | 0.26 | |

| | | | | | | |
|---|---|---|---|---|---|---|
| *Progression 0: negative; Progression 1: positive. ^{#}Staging: in ascending order refers to the progression of breast cancer. | | | | | | |

dPCR analyses for the tested genes provide more accurate, reproducible, and statistically significant results.

### Example 4 - In vitro process for cancer diagnosis and/or prognosis: quantification of HIF-1α and other markers in liquid biopsy using qPCR.

The marker panel data from example 3 was supplemented with the quantitative assessment of HIF-1α and GLUT1, seeking to enable an *in vitro* process for screening, diagnosis and/or prognosis of breast cancer.

This study was conducted according to the protocol available in the article: Diagnostic potential of hypoxia-induced genes in liquid biopsies of breast cancer patients (https://doi.org/10.1038/s41598-021-87897-2). The results can be seen in the tables below.

**Table 9 - Data from the quantification of HIF-1α and RPL13a (reference gene).**

| | **RPL13a** | | | | **HIF1α** | | | |
|---|---|---|---|---|---|---|---|---|
| | **RPL13a Cq** | | | | **HIF1α Cq** | | | |
| **Patient** | **tumor** | **Coll. 1** | **Coll. 2** | **Coll. 3** | **tumor** | **Coll. 1** | **Coll. 2** | **Coll. 3** |
| **1** | 19.07 | 21.7 | 19.5 | 21.3 | NE* | 22.36 | 20.93 | 21.94 |
| **7** | 18.24 | 21.4 | 19.58 | 20 | 27.41 | 22.18 | 21.23 | 21.45 |
| **8** | 18.50 | 23.85 | 19.65 | 23.7 | 23.36 | 24.86 | 20.96 | 23.43 |
| **9** | 31.55 | 18.32 | 19.32 | 21.9 | 40.69 | 20.47 | 20.23 | 22.53 |
| **11** | 27.91 | 21.69 | 19.23 | 18.9 | NE* | 21.75 | 20.13 | 19.83 |
| **12** | 16.75 | 16.03 | 20.22 | 21.1 | 25.09 | 18.90 | 24.41 | 22.68 |
| **13** | 28.03 | 22.45 | 21.26 | 25.5 | 38.55 | 23.69 | 22.71 | 26.59 |
| **15** | 27.71 | 19.70 | 20.4 | 17.5 | 32.53 | 20.59 | 21.56 | 20.34 |
| **16** | 22.24 | 19.10 | 19.15 | 18.5 | 30.51 | 19.39 | 19.84 | 19.86 |
| **17** | 23.47 | 17.69 | 21.41 | 17.55 | 31.24 | 21.78 | 20.72 | 19.80 |
| **18** | 22.48 | 16.12 | 18.96 | 19.49 | 28.53 | 18.60 | 19.74 | 20.31 |
| **21** | 26.81 | 20.91 | 19.92 | 19.91 | 38.72 | 25.22 | 23.07 | 22.26 |
| **22** | 20.32 | 19.94 | 19.29 | 18.82 | 23.76 | 23.32 | 22.55 | 22.35 |
| **24** | 24.32 | 19.80 | 19.50 | 21.31 | 23.59 | 22.94 | 22.01 | 23.90 |
| **25** | 20.02 | 21.06 | 18.91 | 17.31 | 26.14 | 23.03 | 22.05 | 22.41 |
| **26** | 20.20 | 20.90 | 21.04 | 20.18 | 26.62 | 24.76 | 24.69 | 24.47 |
| **27** | 20.81 | 21.03 | 19.59 | 18.65 | 27.82 | 23.59 | 22.57 | 21.91 |
| **28** | 17.91 | 20.29 | 18.27 | 25.01 | 28.63 | 23.74 | 21.35 | 27.04 |
| **29** | 32.39 | 17.98 | 24.25 | 20.18 | NE* | 21.25 | 27.07 | 22.97 |
| **30** | 20.71 | 20.39 | 19.89 | 17.34 | 28.12 | 23.55 | 23.89 | 22.21 |
| **32** | 25.96 | 17.75 | 20.31 | 16.28 | 33.19 | 22.60 | 23.90 | 21.12 |
| **33** | 17.58 | 15.90 | 19.21 | 19.66 | 21.65 | 21.15 | 21.88 | 23.51 |
| **35** | 17.77 | 19.15 | 18.95 | 20.10 | 23.96 | 22.09 | 22.38 | 23.06 |
| **36** | 17.16 | 18.90 | 19.72 | 20.24 | 22.77 | 22.85 | 22.93 | 23.54 |
| **37** | 23.95 | 19.86 | 18.04 | 18.98 | NE* | 23.57 | 22.57 | 23.43 |
| **38** | 21.66 | 18.13 | 18.24 | 24.19 | NE* | 20.85 | 20.73 | 26.06 |
| **39** | 21.53 | 19.88 | 19.79 | 20.39 | 27.06 | 22.25 | 21.30 | 23.23 |
| **40** | 23.60 | | 18.85 | 19.17 | 35.26 | 24.11 | 22.66 | 22.17 |
| **41** | 19.35 | 17.30 | 18.82 | 20.27 | 24.79 | 22.43 | 21.06 | 23.25 |
| **42** | 17.31 | 14.68 | 17.08 | 17.11 | 23.61 | 18.85 | 22.76 | 21.98 |
| **43** | 16.46 | 16.22 | 15.31 | 17.34 | 20.83 | 14.05 | 18.48 | 11.21 |
| **45** | 14.88 | 17.28 | 17.49 | 16.82 | 18.93 | 20.78 | 20.60 | 20.97 |
| **47** | 24.61 | 16.07 | 16.20 | 18.75 | NE* | 22.47 | 20.23 | 19.79 |
| **48** | 19.6604 | 19.13 | 19.67 | 18.81 | 26.44 | | | |
| **49** | 19.61 | 15.25 | 15.72 | 16.22 | 31.99 | 18.60 | 16.77 | 20.32 |
| **50** | 29.13 | 18.33 | 17.28 | 20.61 | 30.47 | | | |
| **52** | 27.94 | 16.54 | 18.80 | 23.19 | NE* | 19.60 | 21.33 | 27.03 |
| **54** | 27.71 | 19.92 | 19.08 | 22.81 | 38.58 | | | |
| **56** | 16.41 | 20.08 | 19.00 | 19.98 | 21.92 | | | |
| **57** | 19.24 | 19.62 | 19.07 | 20.94 | 19.74 | | | |
| **58** | 18.12 | 16.94 | 16.99 | 16.09 | 19.56 | 22.35 | 21.79 | 21.59 |
| **60** | 16.77 | 15.58 | 15.51 | 17.02 | 18.00 | 20.80 | 20.66 | 21.24 |
| **61** | 24.95 | 14.94 | 15.97 | 15.54 | 31.30 | 20.79 | 21.52 | 21.33 |
| **62** | 33.84 | 17.55 | 18.39 | 17.50 | NE* | 23.63 | 22.58 | 21.88 |
| **64** | 20.35 | 17.42 | 18.30 | 17.53 | 22.95 | 22.36 | 24.31 | 21.34 |
| **65** | 16.92 | 17.14 | 18.02 | 19.25 | 17.25 | 22.00 | 22.07 | 21.84 |
| **67** | NE* | 17.15 | 17.95 | 17.96 | NE* | 23.29 | 23.25 | 23.70 |
| **68** | 19.41 | 17.25 | 18.08 | 17.91 | 27.25 | 22.82 | 22.56 | 23.28 |
| **71** | 20.35 | 17.24 | 16.95 | 19.96 | 29.25 | 23.16 | 21.76 | 23.90 |
| **72** | 20.37 | 17.52 | 17.39 | 18.29 | 24.49 | 22.98 | 23.01 | 23.88 |
| **74** | NE* | 19.66 | 18.42 | 17.83 | NE* | 24.21 | 22.55 | 22.21 |
| **76** | 16.39 | 16.04 | 19.13 | 19.76 | 18.12 | 22.70 | 22.46 | 23.10 |
| **78** | 26.07 | 16.51 | 16.86 | 16.80 | NE* | 22.37 | 22.11 | 22.16 |
| **79** | 30.91 | 17.16 | 17.91 | 22.46 | NE* | 22.39 | 22.21 | 27.35 |
| **80** | 22.21 | 17.60 | 20.39 | 20.11 | 26.20 | 22.00 | 24.83 | 28.62 |
| **81** | 17.13 | 18.06 | 18.72 | 20.11 | 19.23 | 22.45 | 26.07 | 23.46 |
| **82** | 22.49 | 15.83 | 21.89 | 15.93 | 25.65 | 19.94 | 25.38 | 18.97 |
| **83** | 27.81 | 18.70 | 19.80 | 18.26 | NE* | | | |
| **84** | 30.64 | 19.45 | 19.21 | 20.88 | NE* | 24.17 | 29.75 | 23.08 |
| **85** | 19.14 | 20.11 | 19.91 | | 20.15 | 24.86 | 22.49 | 19.73 |
| **86** | 17.34 | 16.53 | 19.53 | 17.29 | 26.35 | 17.50 | 23.20 | 23.74 |
| **87** | 16.88 | 20.96 | 24.39 | 21.49 | 18.09 | 25.33 | 27.44 | 37.65 |
| **88** | 24.93 | 17.80 | 16.49 | 19.15 | 43.17 | 25.11 | 20.52 | 22.96 |
| **89** | 25.30 | 16.02 | 16.52 | 18.01 | 32.33 | 20.08 | 20.21 | 21.28 |
| **90** | 20.89 | 16.96 | 16.07 | 17.81 | 32.80 | 20.15 | 19.75 | 13.25 |
| **93** | 23.05 | 15.35 | 20.24 | 19.43 | 28.31 | 18.70 | 22.43 | 19.39 |
| **94** | 20.45 | 19.10 | 20.76 | 18.61 | 24.17 | 21.61 | 22.52 | 19.70 |
| **95** | 24.89 | 23.55 | 22.57 | 22.45 | 19.99 | 23.78 | 22.31 | 22.51 |
| **96** | 18.1449 | 18.59 | 18.80 | 19.83 | 20.51 | 19.70 | 20.29 | 20.75 |
| **97** | 26.4829 | 19.31 | 21.85 | 20.49 | NE* | 21.09 | 20.64 | 22.01 |
| **99** | 15.5852 | 20.62 | 18.62 | 20.01 | 20.64 | 20.59 | 19.38 | 22.34 |
| **101** | 17.467 | 21.99 | 21.67 | 22.34 | 16.03 | 22.49 | 21.26 | 23.19 |
| **102** | | 22.24 | 22.94 | 23.28 | | | | |
| **106** | 18.0169 | 17.74 | 17.73 | 17.18 | 17.60 | 21.89 | 20.75 | 18.79 |
| **107** | 16.2582 | 19.66 | 18.88 | 19.36 | 19.93 | 23.66 | 20.66 | 20.71 |
| **109** | 18.8699 | 21.72 | 22.31 | 27.22 | 27.71 | 21.23 | 22.29 | 26.47 |
| **110** | | 22.54 | 23.63 | 22.98 | | | | |
| **111** | | 24.37 | 22.60 | 25.31 | | | | |
| **112** | | NE* | 24.73 | 23.61 | | | | |
| **114** | | 24.10 | 22.49 | 24.22 | | | | |
| **116** | 18.8703 | 25.39 | 21.95 | 20.46 | 23.76 | 24.72 | 22.92 | 22.28 |
| **117** | 17.9299 | 21.76 | 23.39 | 22.49 | 21.71 | 22.27 | 23.61 | 23.42 |
| **118** | 16.6794 | 20.74 | 21.62 | 22.49 | 16.88 | 21.75 | 21.76 | 22.51 |
| **119** | 16.6881 | 21.01 | 21.74 | 21.58 | 20.40 | 21.17 | 21.78 | 20.56 |
| **120** | 18.744 | 16.46 | 17.47 | 16.98 | 24.56 | 20.56 | 21.40 | 20.79 |
| **121** | 20.5529 | 18.08 | 20.36 | 18.17 | 30.88 | 22.24 | 23.73 | 21.65 |
| **122** | 18.6294 | 19.14 | 16.93 | 19.09 | 23.56 | 22.66 | 20.49 | 22.78 |
| **127** | 23.266 | 17.43 | 18.71 | 18.41 | 34.22 | 20.98 | 21.86 | 22.43 |
| **128** | 18.2624 | 18.88 | 18.68 | 32.87 | 20.37 | 21.51 | 23.31 | NE* |
| **129** | 21.0459 | 20.81 | 20.46 | 19.01 | 42.12 | 24.48 | 23.13 | 22.39 |
| **130** | 20.9885 | 19.85 | 21.20 | 20.29 | 27.23 | 23.00 | 24.25 | 22.80 |
| **131** | 20.9215 | 18.79 | 17.83 | 21.84 | 25.27 | 22.39 | 21.59 | 25.41 |
| **132** | 22.7948 | 23.34 | 18.53 | 22.13 | 25.79 | 27.36 | 22.13 | 25.74 |
| **136** | 15.9291 | 19.22 | 18.95 | 19.68 | 22.88 | 22.83 | 22.83 | 23.78 |
| **137** | 25.2638 | 14.71 | 15.48 | 17.99 | NE* | 22.43 | 22.53 | 23.68 |
| **140** | 19.2509 | 15.66 | 14.41 | 15.00 | 27.11 | 23.11 | 21.00 | 21.71 |
| **141** | 15.952 | 17.06 | 22.92 | 19.47 | NE* | 22.22 | 26.65 | 22.99 |
| **142** | 27.0362 | 17.14 | 15.49 | 16.27 | 39.90 | 23.10 | 20.91 | 23.08 |
| **144** | 24.372 | 18.94 | 18.84 | 20.65 | NE* | 23.08 | 22.67 | 23.93 |
| **145** | 25.4222 | 18.50 | 20.15 | 20.01 | NE* | 23.00 | 23.31 | 24.97 |
| **149** | 16.6286 | 20.70 | 19.29 | 19.04 | 26.64 | 23.88 | 23.85 | 22.88 |
| **150** | 17.5838 | 19.20 | 19.46 | 20.89 | 25.95 | 22.75 | 22.00 | 24.65 |
| **151** | 17.3318 | 17.27 | 20.19 | 18.21 | 20.17 | 22.99 | 24.76 | 22.77 |
| **152** | | 19.95 | 19.14 | 20.90 | | | | |
| **154** | 20.5531 | 18.51 | 18.86 | 19.01 | 29.62 | 21.82 | 22.67 | 23.05 |
| **157** | 19.6881 | 18.72 | 18.48 | 19.77 | 29.53 | 22.26 | 22.15 | 23.19 |
| **158** | 23.7158 | 15.13 | 16.01 | 19.48 | 36.53 | 20.63 | 22.18 | 27.33 |
| **159** | 18.5658 | 15.09 | 17.57 | 16.39 | 15.30 | 20.62 | 24.37 | 23.90 |
| **160** | 23.2303 | 16.57 | 17.28 | 16.17 | 31.95 | 21.83 | 22.32 | 21.33 |
| **163** | 22.4665 | 18.19 | 19.37 | 16.63 | 34.59 | 23.69 | 25.71 | 22.55 |
| **164** | 20.4059 | 15.75 | 18.02 | 15.55 | 25.93 | 23.92 | 24.57 | 21.85 |
| **167** | | 18.71 | 17.99 | 20.98 | | | | |
| **170** | 16.8815 | 20.31 | 22.34 | 20.99 | 22.75 | 26.17 | 26.53 | 25.67 |
| **171** | 15.8441 | 21.77 | 21.85 | 22.15 | 22.16 | 25.17 | 26.19 | 25.73 |
| **173** | 22.4339 | 21.86 | 21.90 | 21.35 | 37.19 | 25.20 | 25.83 | 25.43 |
| **176** | 16.19 | 22.05 | 21.83 | 22.49 | 22.05 | 25.23 | 24.98 | 25.44 |
| **177** | 15.5864 | 21.43 | 21.26 | 21.63 | 21.56 | 25.87 | 25.93 | 26.09 |
| **178** | 22.9975 | 22.12 | 20.27 | 22.05 | NE* | 26.39 | 25.65 | 25.43 |
| **179** | 23.4255 | 21.19 | 23.30 | 20.99 | NE* | 25.45 | 27.31 | 25.13 |
| **184** | 18.9032 | 21.91 | 20.95 | 21.80 | 26.99 | 26.18 | 25.09 | 25.84 |
| **186** | 15.9163 | 21.50 | 20.42 | 20.97 | 23.10 | 26.15 | 25.07 | 28.27 |
| **187** | 23.7256 | 21.62 | 20.99 | 19.78 | 31.21 | 27.00 | 25.55 | 25.16 |
| **191** | 23.6263 | 21.61 | 21.93 | 24.58 | 35.57 | 25.89 | 24.52 | 27.91 |

| | | | | | | | | |
|---|---|---|---|---|---|---|---|---|
| *NE: No expression. | | | | | | | | |

**Table 10 - Data from the quantification of GLUT1 and RPL13a (reference gene).**

| | **RPL13a** | | | | **GLUT1** | | | |
|---|---|---|---|---|---|---|---|---|
| | **RPL13a Cq** | | | | **GLUT1 Cq** | | | |
| **Patient** | **tumor** | **Coll. 1** | **Coll. 2** | **Coll. 3** | **tumor** | **Coll. 1** | **Coll. 2** | **Coll. 3** |
| **1** | 19.07 | 21.7 | 19.5 | 21.3 | NE* | 29.26 | 27.39 | 28.91 |
| **7** | 18.24 | 21.4 | 19.58 | 20 | NE* | 29.04 | 27.49 | 27.41 |
| **8** | 18.50 | 23.85 | 19.65 | 23.7 | 24.61 | 31.32 | 28.05 | 30.42 |
| **9** | 31.55 | 18.32 | 19.32 | 21.9 | NE* | 29.15 | 27.27 | 30.16 |
| **11** | 27.91 | 21.69 | 19.23 | 18.9 | NE* | 28.85 | 25.98 | 25.15 |
| **12** | 16.75 | 16.03 | 20.22 | 21.1 | 37.06 | 27.62 | 30.93 | 28.47 |
| **13** | 28.03 | 22.45 | 21.26 | 25.5 | 31.93 | 29.35 | 28.82 | 33.10 |
| **15** | 27.71 | 19.70 | 20.4 | 17.5 | 31.81 | 27.23 | 27.68 | 28.04 |
| **16** | 22.24 | 19.10 | 19.15 | 18.5 | 33.14 | 26.65 | 27.03 | 28.87 |
| **17** | 23.47 | 17.69 | 21.41 | 17.55 | NE* | 29.95 | 28.63 | 28.91 |
| **18** | 22.48 | 16.12 | 18.96 | 19.49 | NE* | 27.49 | 26.63 | 26.79 |
| **21** | 26.81 | 20.91 | 19.92 | 19.91 | NE* | 26.46 | 25.62 | 24.60 |
| **22** | 20.32 | 19.94 | 19.29 | 18.82 | 27.81 | 25.53 | 24.65 | 23.49 |
| **24** | 24.32 | 19.80 | 19.50 | 21.31 | 33.40 | NE* | NE* | NE* |
| **25** | 20.02 | 21.06 | 18.91 | 17.31 | 32.91 | 25.46 | 24.23 | 25.07 |
| **26** | 20.20 | 20.90 | 21.04 | 20.18 | 29.75 | 25.93 | 26.21 | 25.14 |
| **27** | 20.81 | 21.03 | 19.59 | 18.65 | 30.99 | 25.64 | 24.94 | 23.94 |
| **28** | 17.91 | 20.29 | 18.27 | 25.01 | 31.589 | 26.01 | 23.82 | 30.20 |
| **29** | 32.39 | 17.98 | 24.25 | 20.18 | NE* | 23.07 | 29.44 | 24.81 |
| **30** | 20.71 | 20.39 | 19.89 | 17.34 | 31.11 | 25.29 | 25.31 | 25.57 |
| **32** | 25.96 | 17.75 | 20.31 | 16.28 | NE* | 27.50 | 24.04 | 24.25 |
| **33** | 17.58 | 15.90 | 19.21 | 19.66 | 23.32 | 25.26 | 23.62 | 24.57 |
| **35** | 17.77 | 19.15 | 18.95 | 20.10 | NE* | 25.24 | 24.57 | 25.20 |
| **36** | 17.16 | 18.90 | 19.72 | 20.24 | 25.53 | 24.41 | 25.79 | 26.00 |
| **37** | 23.95 | 19.86 | 18.04 | 18.98 | NE* | 25.71 | 24.34 | 25.46 |
| **38** | 21.66 | 18.13 | 18.24 | 24.19 | NE* | 23.85 | 23.70 | 29.56 |
| **39** | 21.53 | 19.88 | 19.79 | 20.39 | 27.74 | 24.37 | 24.74 | 25.43 |
| **40** | 23.60 | NE* | 18.85 | 19.17 | 33.80 | NE* | 24.50 | 24.93 |
| **41** | 19.35 | 17.30 | 18.82 | 20.27 | 26.26 | 27.35 | 24.67 | 26.14 |
| **42** | 17.31 | 14.68 | 17.08 | 17.11 | NE* | 25.23 | 28.54 | 26.34 |
| **43** | 16.46 | 16.22 | 15.31 | 17.34 | 25.19 | 25.53 | 24.06 | 25.69 |
| **45** | 14.88 | 17.28 | 17.49 | 16.82 | 24.32 | 25.05 | 25.49 | 26.02 |
| **47** | 24.61 | 16.07 | 16.20 | 18.75 | 32.59 | 25.91 | 25.75 | 27.41 |
| **48** | 19.6604 | 19.13 | 19.67 | 18.81 | NE* | NE* | NE* | NE* |
| **49** | 19.61 | 15.25 | 15.72 | 16.22 | 32.82 | 25.10 | 23.90 | 24.71 |
| **50** | 29.13 | 18.33 | 17.28 | 20.61 | 32.29 | 25.86 | 24.21 | 26.33 |
| **52** | 27.94 | 16.54 | 18.80 | 23.19 | NE* | 26.54 | 29.29 | 31.60 |
| **54** | 27.71 | 19.92 | 19.08 | 22.81 | NE* | 26.53 | 26.92 | 27.94 |
| **56** | 16.41 | 20.08 | 19.00 | 19.98 | 22.13 | 27.55 | 26.42 | 25.98 |
| **57** | 19.24 | 19.62 | 19.07 | 20.94 | NE* | NE* | 25.76 | 26.70 |
| **58** | 18.12 | 16.94 | 16.99 | 16.09 | 23.92 | 27.13 | 27.08 | 26.62 |
| **60** | 16.77 | 15.58 | 15.51 | 17.02 | 22.46 | 27.49 | 27.36 | 27.60 |
| **61** | 24.95 | 14.94 | 15.97 | 15.54 | 32.97 | 27.80 | 26.84 | 27.72 |
| **62** | 33.84 | 17.55 | 18.39 | 17.50 | NE* | 28.02 | 27.63 | 26.79 |
| **64** | 20.35 | 17.42 | 18.30 | 17.53 | 34.03 | 26.64 | 35.33 | 26.26 |
| **65** | 16.92 | 17.14 | 18.02 | 19.25 | 27.72 | 28.05 | 27.46 | 28.21 |
| **67** | NE* | 17.15 | 17.95 | 17.96 | NE* | 27.33 | 27.93 | 27.27 |
| **68** | 19.41 | 17.25 | 18.08 | 17.91 | NE* | 28.33 | 29.68 | 28.45 |
| **71** | 20.35 | 17.24 | 16.95 | 19.96 | 36.10 | 29.92 | 28.32 | 30.36 |
| **72** | 20.37 | 17.52 | 17.39 | 18.29 | 34.06 | 32.38 | 29.32 | 29.71 |
| **74** | NE* | 19.66 | 18.42 | 17.83 | NE* | 30.49 | 29.36 | 28.50 |
| **76** | 16.39 | 16.04 | 19.13 | 19.76 | 28.32 | 29.60 | 29.13 | 29.65 |
| **78** | 26.07 | 16.51 | 16.86 | 16.80 | NE* | 27.20 | 28.57 | 27.85 |
| **79** | 30.91 | 17.16 | 17.91 | 22.46 | NE* | 28.38 | 27.33 | 32.01 |
| **80** | 22.21 | 17.60 | 20.39 | 20.11 | 34.83 | 28.00 | 31.42 | 29.83 |
| **81** | 17.13 | 18.06 | 18.72 | 20.11 | 31.93 | 27.14 | 29.56 | 26.95 |
| **82** | 22.49 | 15.83 | 21.89 | 15.93 | NE* | 29.72 | 30.56 | 24.98 |
| **83** | 27.81 | 18.70 | 19.80 | 18.26 | NE* | 27.29 | 27.06 | 25.89 |
| **84** | 30.64 | 19.45 | 19.21 | 20.88 | NE* | 26.10 | 25.88 | 25.35 |
| **85** | 19.14 | 20.11 | 19.91 | NE* | 34.95 | 26.81 | 24.52 | 22.41 |
| **86** | 17.34 | 16.53 | 19.53 | 17.29 | 35.63 | 21.99 | 25.88 | 26.54 |
| **87** | 16.88 | 20.96 | 24.39 | 21.49 | 29.55 | 27.96 | 29.88 | 28.88 |
| **88** | 24.93 | 17.80 | 16.49 | 19.15 | NE* | 23.96 | 23.94 | 26.30 |
| **89** | 25.30 | 16.02 | 16.52 | 18.01 | NE* | 23.70 | 23.48 | 22.97 |
| **90** | 20.89 | 16.96 | 16.07 | 17.81 | NE* | 25.26 | 24.35 | 23.00 |
| **93** | 23.05 | 15.35 | 20.24 | 19.43 | 37.07 | 23.43 | 26.54 | 22.26 |
| **94** | 20.45 | 19.10 | 20.76 | 18.61 | 37.03 | 25.35 | 25.22 | 23.87 |
| **95** | 24.89 | 23.55 | 22.57 | 22.45 | NE* | 23.39 | 22.36 | 22.67 |
| **96** | 18.1449 | 18.59 | 18.80 | 19.83 | 30.93 | 23.00 | 25.57 | 24.49 |
| **97** | 26.4829 | 19.31 | 21.85 | 20.49 | 39.17 | 31.76 | 31.46 | 32.24 |
| **99** | 15.5852 | 20.62 | 18.62 | 20.01 | NE* | 31.18 | 30.49 | 33.19 |
| **101** | 17.467 | 21.99 | 21.67 | 22.34 | 28.19 | 29.34 | 28.99 | 29.30 |
| **102** | NE* | 22.24 | 22.94 | 23.28 | NE* | 28.87 | 29.79 | 30.15 |
| **106** | 18.0169 | 17.74 | 17.73 | 17.18 | 28.89 | 31.67 | 35.44 | 28.41 |
| **107** | 16.2582 | 19.66 | 18.88 | 19.36 | 28.49 | 32.80 | 29.16 | 30.05 |
| **109** | 18.8699 | 21.72 | 22.31 | 27.22 | 38.37 | 28.96 | 29.95 | 34.08 |
| **110** | NE* | 22.54 | 23.63 | 22.98 | NE* | 29.82 | 31.60 | 30.39 |
| **111** | NE* | 24.37 | 22.60 | 25.31 | NE* | 31.34 | 30.10 | 31.88 |
| **112** | NE* | NE* | 24.73 | 23.61 | NE* | 30.12 | 32.43 | 30.80 |
| **114** | NE* | 24.10 | 22.49 | 24.22 | NE* | 31.11 | 29.34 | 27.70 |
| **116** | 18.8703 | 25.39 | 21.95 | 20.46 | NE* | 27.75 | 26.43 | 26.39 |
| **117** | 17.9299 | 21.76 | 23.39 | 22.49 | 36.09 | 25.83 | 28.00 | 26.25 |
| **118** | 16.6794 | 20.74 | 21.62 | 22.49 | 27.80 | 25.51 | 26.88 | 26.23 |
| **119** | 16.6881 | 21.01 | 21.74 | 21.58 | 30.18 | 24.79 | 25.48 | 24.88 |
| **120** | 18.744 | 16.46 | 17.47 | 16.98 | 36.19 | 24.47 | 25.69 | 25.07 |
| **121** | 20.5529 | 18.08 | 20.36 | 18.17 | NE* | 25.62 | 27.63 | 24.68 |
| **122** | 18.6294 | 19.14 | 16.93 | 19.09 | 38.12 | 26.68 | 25.08 | 26.39 |
| **127** | 23.266 | 17.43 | 18.71 | 18.41 | NE* | 25.57 | 26.78 | 26.95 |
| **128** | 18.2624 | 18.88 | 18.68 | 32.87 | 31.00 | 26.92 | 28.28 | NE* |
| **129** | 21.0459 | 20.81 | 20.46 | 19.01 | 43.12 | 28.52 | 27.72 | 26.74 |
| **130** | 20.9885 | 19.85 | 21.20 | 20.29 | 35.60 | 27.38 | 28.40 | 27.71 |
| **131** | 20.9215 | 18.79 | 17.83 | 21.84 | 35.06 | 27.45 | 26.75 | 29.58 |
| **132** | 22.7948 | 23.34 | 18.53 | 22.13 | 37.73 | 32.06 | 27.06 | 29.98 |
| **136** | 15.9291 | 19.22 | 18.95 | 19.68 | NE* | 27.46 | 26.83 | 27.27 |
| **137** | 25.2638 | 14.71 | 15.48 | 17.99 | 37.54 | 27.30 | 29.41 | 27.42 |
| **140** | 19.2509 | 15.66 | 14.41 | 15.00 | 32.39 | 27.63 | 25.25 | 27.00 |
| **141** | 15.952 | 17.06 | 22.92 | 19.47 | NE* | 24.91 | 29.48 | 26.56 |
| **142** | 27.0362 | 17.14 | 15.49 | 16.27 | NE* | 27.41 | 24.72 | 26.87 |
| **144** | 24.372 | 18.94 | 18.84 | 20.65 | 39.62 | 26.09 | 26.03 | 27.80 |
| **145** | 25.4222 | 18.50 | 20.15 | 20.01 | 38.87 | 25.56 | 26.37 | 26.19 |
| **149** | 16.6286 | 20.70 | 19.29 | 19.04 | 31.12 | 26.83 | 26.86 | 26.36 |
| **150** | 17.5838 | 19.20 | 19.46 | 20.89 | 33.62 | 26.36 | 26.55 | 26.89 |
| **151** | 17.3318 | 17.27 | 20.19 | 18.21 | 26.99 | 25.00 | 27.82 | 25.86 |
| **152** | NE* | 19.95 | 19.14 | 20.90 | NE* | 27.64 | 26.26 | 28.37 |
| **154** | 20.5531 | 18.51 | 18.86 | 19.01 | NE* | 24.73 | 25.73 | 25.60 |
| **157** | 19.6881 | 18.72 | 18.48 | 19.77 | 36.07 | 25.60 | 25.52 | 26.73 |
| **158** | 23.7158 | 15.13 | 16.01 | 19.48 | NE* | 25.69 | 28.43 | 32.91 |
| **159** | 18.5658 | 15.09 | 17.57 | 16.39 | NE* | 25.81 | 28.86 | 33.20 |
| **160** | 23.2303 | 16.57 | 17.28 | 16.17 | NE* | 28.37 | 27.71 | 27.07 |
| **163** | 22.4665 | 18.19 | 19.37 | 16.63 | NE* | 30.24 | 29.93 | 26.55 |
| **164** | 20.4059 | 15.75 | 18.02 | 15.55 | NE* | 24.44 | 28.59 | 26.94 |
| **167** | NE* | 18.71 | 17.99 | 20.98 | NE* | 28.99 | 29.68 | 30.65 |
| **170** | 16.8815 | 20.31 | 22.34 | 20.99 | 28.84 | 27.61 | 28.85 | 27.40 |
| **171** | 15.8441 | 21.77 | 21.85 | 22.15 | NE* | 28.00 | 29.54 | 29.34 |
| **173** | 22.4339 | 21.86 | 21.90 | 21.35 | NE* | 28.21 | 28.47 | 28.79 |
| **176** | 16.19 | 22.05 | 21.83 | 22.49 | 27.05 | 29.46 | 28.81 | 29.33 |
| **177** | 15.5864 | 21.43 | 21.26 | 21.63 | 28.30 | 29.38 | 29.16 | 29.48 |
| **178** | 22.9975 | 22.12 | 20.27 | 22.05 | NE* | 29.61 | 28.29 | 28.09 |
| **179** | 23.4255 | 21.19 | 23.30 | 20.99 | NE* | 28.08 | 30.73 | 28.00 |
| **184** | 18.9032 | 21.91 | 20.95 | 21.80 | 36.09 | 29.81 | 28.78 | 28.50 |
| **186** | 15.9163 | 21.50 | 20.42 | 20.97 | 31.92 | 29.52 | 28.56 | 28.08 |
| **187** | 23.7256 | 21.62 | 20.99 | 19.78 | 37.82 | 28.61 | 28.18 | 27.96 |
| **191** | 23.6263 | 21.61 | 21.93 | 24.58 | NE* | 29.16 | 29.08 | 31.13 |

| | | | | | | | | |
|---|---|---|---|---|---|---|---|---|
| *NE: No expression. | | | | | | | | |

Using the approach described in example 3, the values of *HIF-1α, 2^-*(Δ Cq) and *GLUT1,* 2^-(Δ Cq) were calculated. Tables 11 and 12 show the respective values.

**Table 11 - HIF-1α 2^-(ΔCq) values, progression and staging.**

| | | | **HIF1a 2^-(ΔCq)** | | | | |
|---|---|---|---|---|---|---|---|
| **patient** | **Progression*** | **Staging^{#}** | **donor** | **tumor** | **coll. 1** | **coll. 2** | **coll. 3** |
| 1 | 0 | 1 | 0.001 | | 0.466 | 0.377 | 0.627 |
| 7 | 1 | 2 | 0.004 | 0.002 | 0.602 | 0.318 | 0.361 |
| 8 | 0 | 2 | 0.002 | 0.034 | 0.496 | 0.403 | |
| 9 | 0 | 1 | 0.001 | 0.002 | 0.226 | 0.532 | 0.667 |
| 11 | 0 | 2 | 0.000 | | 0.958 | 0.535 | 0.513 |
| 12 | 0 | 2 | 0.001 | 0.003 | 0.136 | 0.055 | 0.328 |
| 13 | 0 | 3 | 0.001 | 0.001 | 0.425 | 0.366 | 0.477 |
| 15 | 0 | 2 | 0.000 | 0.035 | 0.537 | 0.449 | 0.143 |
| 16 | 0 | 2 | 0.001 | 0.003 | 0.822 | 0.619 | 0.377 |
| 17 | 1 | 2 | 0.001 | 0.005 | 0.059 | 1.607 | 0.210 |
| 18 | 0 | 1 | 0.000 | 0.015 | 0.180 | 0.582 | 0.569 |
| 21 | 0 | 2 | 0.005 | 0.000 | 0.050 | 0.112 | 0.196 |
| 22 | 0 | 2 | 0.005 | 0.092 | 0.096 | 0.105 | 0.086 |
| 24 | 0 | 1 | 0.004 | | 0.114 | 0.175 | 0.166 |
| 25 | 0 | 3 | 0.003 | 0.014 | 0.255 | 0.114 | 0.029 |
| 26 | 0 | 2 | 0.002 | 0.012 | 0.069 | 0.079 | 0.051 |
| 27 | 0 | 3 | 0.001 | 0.008 | 0.169 | 0.127 | 0.104 |
| 28 | 1 | 3 | 0.002 | 0.001 | 0.092 | 0.119 | 0.245 |
| 29 | 0 | 1 | 0.002 | | 0.103 | 0.142 | 0.145 |
| 30 | 0 | 1 | 0.002 | 0.006 | 0.112 | 0.063 | 0.034 |
| 32 | 0 | 1 | 0.002 | 0.007 | 0.035 | 0.083 | 0.035 |
| 33 | 0 | 3 | 0.004 | 0.060 | 0.026 | 0.158 | 0.069 |
| 35 | 0 | 1 | 0.002 | 0.014 | 0.130 | 0.093 | 0.129 |
| 36 | 0 | 3 | 0.003 | 0.020 | 0.065 | 0.108 | 0.102 |
| 37 | 0 | 3 | 0.004 | | 0.076 | 0.043 | 0.046 |
| 38 | 0 | 2 | | | 0.152 | 0.178 | 0.273 |
| 39 | 0 | 3 | | 0.022 | 0.193 | 0.352 | 0.139 |
| 40 | 0 | 3 | | 0.000 | 0.000 | 0.071 | 0.125 |
| 41 | 0 | 3 | | 0.023 | 0.028 | 0.212 | 0.127 |
| 42 | 0 | 2 | | 0.013 | 0.055 | 0.019 | 0.034 |
| 43 | 0 | 1 | | 0.048 | 4.505 | 0.111 | |
| 45 | 0 | 2 | | 0.060 | 0.088 | 0.116 | 0.057 |
| 47 | 0 | 3 | | | 0.012 | 0.061 | 0.486 |
| 48 | 0 | 3 | | 0.009 | | | |
| 49 | 0 | 2 | | 0.000 | 0.098 | 0.483 | 0.058 |
| 50 | 0 | 3 | | 0.396 | | | |
| 52 | 0 | 1 | | | 0.120 | 0.174 | 0.069 |
| 54 | 0 | 2 | | 0.001 | | | |
| 56 | 0 | 2 | | 0.022 | | | |
| 57 | 0 | 1 | | 0.707 | | | |
| 58 | 0 | 3 | | 0.369 | 0.024 | 0.036 | 0.022 |
| 60 | 0 | 3 | | 0.426 | 0.027 | 0.028 | 0.054 |
| 61 | 1 | 3 | | 0.012 | 0.017 | 0.021 | 0.018 |
| 62 | 0 | 2 | | | 0.015 | 0.055 | 0.048 |
| 64 | 0 | 3 | | 0.165 | 0.033 | 0.016 | 0.071 |
| 65 | 0 | 1 | | 0.792 | 0.035 | 0.060 | 0.166 |
| 67 | 0 | 2 | | | 0.014 | 0.025 | 0.019 |
| 68 | 0 | 2 | | 0.004 | 0.021 | 0.045 | 0.024 |
| 71 | 0 | 2 | | 0.002 | 0.017 | 0.036 | 0.065 |
| 72 | 0 | 3 | | 0.058 | 0.023 | 0.020 | 0.021 |
| 74 | 0 | 2 | | | 0.042 | 0.057 | 0.048 |
| 76 | 1 | 2 | | 0.302 | 0.010 | 0.099 | 0.099 |
| 78 | | | | | 0.017 | 0.026 | 0.024 |
| 79 | 0 | 2 | | | 0.027 | 0.051 | 0.034 |
| 80 | 0 | 2 | | 0.063 | 0.047 | 0.046 | 0.003 |
| 81 | 0 | 2 | | 0.232 | 0.048 | 0.006 | 0.098 |
| 82 | 0 | 2 | | 0.112 | 0.058 | 0.089 | 0.121 |
| 83 | 0 | 2 | | | | | |
| 84 | 0 | 2 | | | 0.038 | 0.001 | 0.219 |
| 85 | 1 | 2 | | 0.498 | 0.037 | 0.167 | |
| 86 | 1 | 3 | | 0.002 | 0.510 | 0.078 | 0.011 |
| 87 | 0 | 3 | | 0.433 | 0.049 | 0.121 | 0.000 |
| 88 | 0 | 2 | | 0.000 | 0.006 | 0.061 | 0.071 |
| 89 | 1 | 3 | | 0.008 | 0.060 | 0.078 | 0.104 |
| 90 | 0 | 3 | | 0.000 | 0.109 | 0.078 | |
| 93 | 0 | 1 | | 0.026 | 0.098 | 0.219 | 1.028 |
| 94 | 0 | 1 | | 0.076 | 0.176 | 0.297 | 0.470 |
| 95 | 0 | 1 | | | 0.850 | 1.192 | 0.965 |
| 96 | 0 | 2 | | 0.195 | 0.462 | 0.356 | 0.528 |
| 97 | 0 | 1 | | | 0.291 | 2.304 | 0.348 |
| 99 | 0 | 1 | | 0.030 | 1.016 | 0.587 | 0.199 |
| 101 | 1 | 3 | | | 0.710 | 1.329 | 0.552 |
| 102 | 0 | 3 | | | | | |
| 106 | 0 | | | 1.331 | 0.057 | 0.123 | 0.329 |
| 107 | 0 | 2 | | 0.078 | 0.062 | 0.292 | 0.393 |
| 109 | 0 | 2 | | 0.002 | | 1.016 | |
| 110 | 0 | 2 | | | | | |
| 111 | 0 | 1 | | | | | |
| 112 | 0 | 2 | | | | | |
| 114 | 0 | 2 | | | | | |
| 116 | 0 | 2 | | 0.034 | | 0.511 | 0.282 |
| 117 | 0 | 2 | | 0.073 | 0.701 | 0.860 | 0.525 |
| 118 | | | | 0.873 | 0.499 | 0.909 | 0.985 |
| 119 | 0 | 2 | | 0.076 | 0.898 | 0.970 | |
| 120 | 0 | 2 | | 0.018 | 0.058 | 0.066 | 0.071 |
| 121 | 0 | 1 | | 0.001 | 0.056 | 0.097 | 0.090 |
| 122 | 0 | 2 | | 0.033 | 0.088 | 0.084 | 0.077 |
| 127 | 0 | 2 | | 0.001 | 0.086 | 0.113 | 0.062 |
| 128 | 0 | 3 | | 0.233 | 0.161 | 0.040 | |
| 129 | 0 | 1 | | 0.000 | 0.079 | 0.158 | 0.096 |
| 130 | 0 | 2 | | 0.013 | 0.113 | 0.121 | 0.176 |
| 131 | 0 | 2 | | 0.049 | 0.083 | 0.074 | 0.084 |
| 132 | 0 | 1 | | 0.125 | 0.062 | 0.082 | 0.082 |
| 136 | 0 | 2 | | 0.008 | 0.082 | 0.068 | 0.058 |
| 137 | 0 | 2 | | | 0.005 | 0.008 | 0.019 |
| 140 | 0 | 2 | | 0.004 | 0.006 | 0.010 | 0.009 |
| 141 | 0 | 2 | | | 0.028 | 0.075 | 0.088 |
| 142 | 0 | 3 | | 0.000 | 0.016 | 0.023 | 0.009 |
| 144 | 0 | | | | 0.057 | 0.070 | 0.103 |
| 145 | 0 | 2 | | | 0.044 | 0.112 | 0.032 |
| 149 | 0 | 2 | | 0.001 | 0.110 | 0.042 | 0.070 |
| 150 | 0 | 2 | | 0.003 | 0.086 | 0.173 | 0.074 |
| 151 | 0 | 1 | | 0.140 | 0.019 | 0.042 | 0.042 |
| 152 | 0 | 3 | | | | | |
| 154 | 0 | 3 | | 0.002 | 0.101 | 0.071 | 0.061 |
| 157 | 0 | | | 0.001 | 0.086 | 0.078 | 0.094 |
| 158 | 0 | 2 | | 0.000 | 0.022 | 0.014 | 0.004 |
| 159 | 0 | 1 | | | 0.022 | 0.009 | 0.005 |
| 160 | 0 | 1 | | 0.002 | 0.026 | 0.030 | 0.028 |
| 163 | 0 | 2 | | 0.000 | 0.022 | 0.012 | 0.016 |
| 164 | 0 | 1 | | 0.022 | 0.003 | 0.011 | 0.013 |
| 167 | 0 | 3 | | | | | |
| 168 | 0 | 1 | | | | | |
| 169 | 0 | | | | | | |
| 170 | 0 | 1 | | 0.017 | 0.017 | 0.055 | 0.039 |
| 171 | 1 | 2 | | 0.013 | 0.094 | 0.049 | 0.084 |
| 173 | 0 | 2 | | 0.000 | 0.099 | 0.066 | 0.059 |
| 176 | 0 | 2 | | 0.017 | 0.110 | 0.113 | 0.130 |
| 177 | 0 | 2 | | 0.016 | 0.046 | 0.039 | 0.045 |
| 178 | 0 | 2 | | | 0.051 | 0.024 | 0.096 |
| 179 | 0 | 2 | | | 0.052 | 0.062 | 0.057 |
| 184 | 0 | 2 | | 0.004 | 0.052 | 0.057 | 0.061 |
| 186 | 0 | 0 | | 0.007 | 0.040 | 0.040 | 0.006 |
| 187 | 0 | 1 | | 0.006 | 0.024 | 0.042 | 0.024 |
| 191 | 0 | 3 | | 0.000 | 0.051 | 0.166 | 0.099 |

| | | | | | | | |
|---|---|---|---|---|---|---|---|
| *Progression 0: negative; Progression 1: positive. ^{#}Staging: in ascending order refers to the progression of breast cancer. | | | | | | | |

**Table 12 - GLUT1 2^-(ΔCq) values, progression and staging.**

| | | | **GLUT1 2^-(ΔCq)** | | | | |
|---|---|---|---|---|---|---|---|
| **patient** | **Progression*** | **Staging^{#}** | **donor** | **tumor** | **coll. 1** | **coll. 2** | **coll. 3** |
| 1 | 0 | 1 | 0.0002 | | 0.004 | 0.004 | 0.005 |
| 7 | 1 | 2 | 0.0001 | | 0.005 | 0.004 | 0.006 |
| 8 | 0 | 2 | 0.0001 | 0.014 | 0.006 | 0.003 | 0.010 |
| 9 | 0 | 1 | 0.0000 | | 0.001 | 0.004 | 0.003 |
| 11 | 0 | 2 | 0.0000 | | 0.007 | 0.009 | 0.013 |
| 12 | 0 | 2 | 0.0000 | 0.000 | 0.000 | 0.001 | 0.006 |
| 13 | 0 | 3 | 0.0000 | 0.067 | 0.008 | 0.005 | 0.005 |
| 15 | 0 | 2 | | 0.058 | 0.005 | 0.006 | 0.001 |
| 16 | 0 | 2 | 0.0001 | 0.001 | 0.005 | 0.004 | 0.001 |
| 17 | 1 | 2 | | | 0.000 | 0.007 | 0.000 |
| 18 | 0 | 1 | 0.0000 | | 0.000 | 0.005 | 0.006 |
| 21 | 0 | 2 | 0.0000 | | 0.021 | 0.019 | 0.039 |
| 22 | 0 | 2 | 0.0002 | 0.006 | 0.021 | 0.024 | 0.039 |
| 24 | 0 | 1 | 0.0001 | 0.002 | | | |
| 25 | 0 | 3 | 0.0001 | 0.000 | 0.048 | 0.025 | 0.005 |
| 26 | 0 | 2 | 0.0000 | 0.001 | 0.031 | 0.028 | 0.032 |
| 27 | 0 | 3 | 0.0000 | 0.001 | 0.041 | 0.025 | 0.026 |
| 28 | 1 | 3 | 0.0000 | 0.000 | 0.019 | 0.021 | 0.027 |
| 29 | 0 | 1 | 0.0000 | | 0.029 | 0.027 | 0.040 |
| 30 | 0 | 1 | | 0.001 | 0.033 | 0.023 | 0.003 |
| 32 | 0 | 1 | 0.0001 | | 0.001 | 0.075 | 0.004 |
| 33 | 0 | 3 | | 0.019 | 0.002 | 0.047 | 0.033 |
| 35 | 0 | 1 | 0.0001 | | 0.015 | 0.020 | 0.029 |
| 36 | 0 | 3 | 0.0001 | 0.003 | 0.022 | 0.015 | 0.018 |
| 37 | 0 | 3 | | | 0.017 | 0.013 | 0.011 |
| 38 | 0 | 2 | | | 0.019 | 0.023 | 0.024 |
| 39 | 0 | 3 | | 0.014 | 0.044 | 0.032 | 0.030 |
| 40 | 0 | 3 | | 0.001 | | 0.020 | 0.018 |
| 41 | 0 | 3 | | 0.008 | 0.001 | 0.017 | 0.017 |
| 42 | 0 | 2 | | | 0.001 | 0.000 | 0.002 |
| 43 | 0 | 1 | | 0.002 | 0.002 | 0.002 | 0.003 |
| 45 | 0 | 2 | | 0.001 | 0.005 | 0.004 | 0.002 |
| 47 | 0 | 3 | | 0.004 | 0.001 | 0.001 | 0.002 |
| 48 | 0 | 3 | | | | | |
| 49 | 0 | 2 | | 0.000 | 0.001 | 0.003 | 0.003 |
| 50 | 0 | 3 | | 0.112 | 0.005 | 0.008 | 0.019 |
| 52 | 0 | 1 | | | 0.001 | 0.001 | 0.003 |
| 54 | 0 | 2 | | | 0.010 | 0.004 | 0.029 |
| 56 | 0 | 2 | | 0.019 | 0.006 | 0.006 | 0.016 |
| 57 | 0 | 1 | | | | 0.010 | 0.018 |
| 58 | 0 | 3 | | 0.018 | 0.001 | 0.001 | 0.001 |
| 60 | 0 | 3 | | 0.019 | 0.000 | 0.000 | 0.001 |
| 61 | 1 | 3 | | 0.004 | 0.000 | 0.001 | 0.000 |
| 62 | 0 | 2 | | | 0.001 | 0.002 | 0.002 |
| 64 | 0 | 3 | | 0.000 | 0.002 | 0.000 | 0.002 |
| 65 | 0 | 1 | | 0.001 | 0.001 | 0.001 | 0.002 |
| 67 | 0 | 2 | | | 0.001 | 0.001 | 0.002 |
| 68 | 0 | 2 | | | 0.000 | 0.000 | 0.001 |
| 71 | 0 | 2 | | 0.000 | 0.000 | 0.000 | 0.001 |
| 72 | 0 | 3 | | 0.000 | 0.000 | 0.000 | 0.000 |
| 74 | 0 | 2 | | | 0.001 | 0.001 | 0.001 |
| 76 | 1 | 2 | | 0.000 | 0.000 | 0.001 | 0.001 |
| 78 | | | | | 0.001 | 0.000 | 0.000 |
| 79 | 0 | 2 | | | 0.000 | 0.001 | 0.001 |
| 80 | 0 | 2 | | 0.000 | 0.001 | 0.000 | 0.001 |
| 81 | 0 | 2 | | 0.000 | 0.002 | 0.001 | 0.009 |
| 82 | 0 | 2 | | | 0.000 | 0.002 | 0.002 |
| 83 | 0 | 2 | | | 0.003 | 0.007 | 0.005 |
| 84 | 0 | 2 | | | 0.010 | 0.010 | 0.045 |
| 85 | 1 | 2 | | 0.000 | 0.010 | 0.041 | |
| 86 | 1 | 3 | | 0.000 | 0.023 | 0.012 | 0.002 |
| 87 | 0 | 3 | | 0.000 | 0.008 | 0.022 | 0.006 |
| 88 | 0 | 2 | | | 0.014 | 0.006 | 0.007 |
| 89 | 1 | 3 | | | 0.005 | 0.008 | 0.032 |
| 90 | 0 | 3 | | | 0.003 | 0.003 | 0.027 |
| 93 | 0 | 1 | | 0.000 | 0.004 | 0.013 | 0.141 |
| 94 | 0 | 1 | | 0.000 | 0.013 | 0.046 | 0.026 |
| 95 | 0 | 1 | | | | | |
| 96 | 0 | 2 | | 0.000 | 0.047 | 0.009 | 0.039 |
| 97 | 0 | 1 | | 0.000 | 0.000 | 0.001 | 0.000 |
| 99 | 0 | 1 | | | 0.001 | 0.000 | 0.000 |
| 101 | 1 | 3 | | 0.001 | 0.006 | 0.006 | 0.008 |
| 102 | 0 | 3 | | | 0.010 | 0.009 | 0.009 |
| 106 | 0 | | | 0.001 | 0.000 | 0.000 | 0.000 |
| 107 | 0 | 2 | | 0.000 | 0.000 | 0.001 | 0.001 |
| 109 | 0 | 2 | | 0.000 | 0.007 | 0.005 | 0.009 |
| 110 | 0 | 2 | | | 0.006 | 0.004 | 0.006 |
| 111 | 0 | 1 | | | 0.008 | 0.006 | 0.011 |
| 112 | 0 | 2 | | | | 0.005 | 0.007 |
| 114 | 0 | 2 | | | 0.008 | 0.009 | 0.089 |
| 116 | 0 | 2 | | | 0.195 | 0.045 | 0.016 |
| 117 | 0 | 2 | | 0.000 | 0.059 | 0.041 | 0.074 |
| 118 | | | | 0.000 | 0.037 | 0.026 | 0.075 |
| 119 | 0 | 2 | | 0.000 | 0.073 | 0.075 | 0.102 |
| 120 | 0 | 2 | | 0.000 | 0.004 | 0.003 | 0.004 |
| 121 | 0 | 1 | | | 0.005 | 0.007 | 0.011 |
| 122 | 0 | 2 | | 0.000 | 0.005 | 0.004 | 0.006 |
| 127 | 0 | 2 | | | 0.004 | 0.004 | 0.003 |
| 128 | 0 | 3 | | 0.000 | 0.004 | 0.001 | |
| 129 | 0 | 1 | | 0.000 | 0.005 | 0.007 | 0.005 |
| 130 | 0 | 2 | | 0.000 | 0.005 | 0.007 | 0.006 |
| 131 | 0 | 2 | | 0.000 | 0.002 | 0.002 | 0.005 |
| 132 | 0 | 1 | | 0.000 | 0.002 | 0.003 | 0.004 |
| 136 | 0 | 2 | | | 0.003 | 0.004 | 0.005 |
| 137 | 0 | 2 | | 0.000 | 0.000 | 0.000 | 0.001 |
| 140 | 0 | 2 | | 0.000 | 0.000 | 0.001 | 0.000 |
| 141 | 0 | 2 | | | 0.004 | 0.011 | 0.007 |
| 142 | 0 | 3 | | | 0.001 | 0.002 | 0.001 |
| 144 | 0 | | | 0.000 | 0.007 | 0.007 | 0.007 |
| 145 | 0 | 2 | | 0.000 | 0.007 | 0.013 | 0.014 |
| 149 | 0 | 2 | | 0.000 | 0.014 | 0.005 | 0.006 |
| 150 | 0 | 2 | | 0.000 | 0.007 | 0.007 | 0.016 |
| 151 | 0 | 1 | | 0.001 | 0.005 | 0.005 | 0.005 |
| 152 | 0 | 3 | | | 0.005 | 0.007 | 0.006 |
| 154 | 0 | 3 | | | 0.013 | 0.009 | 0.010 |
| 157 | 0 | | | 0.000 | 0.008 | 0.008 | 0.008 |
| 158 | 0 | 2 | | | 0.001 | 0.000 | 0.000 |
| 159 | 0 | 1 | | | 0.001 | 0.000 | 0.000 |
| 160 | 0 | 1 | | | 0.000 | 0.001 | 0.001 |
| 163 | 0 | 2 | | | 0.000 | 0.001 | 0.001 |
| 164 | 0 | 1 | | | 0.002 | 0.001 | 0.000 |
| 167 | 0 | 3 | | | 0.001 | 0.000 | 0.001 |
| 168 | 0 | 1 | | | | | |
| 169 | 0 | | | | | | |
| 170 | 0 | 1 | | 0.000 | 0.006 | 0.011 | 0.012 |
| 171 | 1 | 2 | | | 0.013 | 0.005 | 0.007 |
| 173 | 0 | 2 | | | 0.012 | 0.011 | 0.006 |
| 176 | 0 | 2 | | 0.001 | 0.006 | 0.008 | 0.009 |
| 177 | 0 | 2 | | 0.000 | 0.004 | 0.004 | 0.004 |
| 178 | 0 | 2 | | | 0.006 | 0.004 | 0.015 |
| 179 | 0 | 2 | | | 0.008 | 0.006 | 0.008 |
| 184 | 0 | 2 | | 0.000 | 0.004 | 0.004 | 0.010 |
| 186 | 0 | 0 | | 0.000 | 0.004 | 0.004 | 0.007 |
| 187 | 0 | 1 | | 0.000 | 0.008 | 0.007 | 0.003 |
| 191 | 0 | 3 | | | 0.005 | 0.007 | 0.011 |

| | | | | | | | |
|---|---|---|---|---|---|---|---|
| *Progression 0: negative; Progression 1: positive. ^{#}Staging: in ascending order refers to the progression of breast cancer. | | | | | | | |

**Table 13 - Raw Cq values and expression of target markers in different breast tissue cell lines: healthy (MCF-10A), with non-invasive tumor (MCF-7) and with metastatic tumor (MDA-MB-231).**

| **Cell** | **RPL13a** Cq | **GLUT1** Cq | **GLUT1** 2^-(ΔCq) | **HIF-1a** Cq | **HIF-1a** 2^-(ΔCq) | **CAIX** Cq | **CAIX** 2^-(ΔCq) |
|---|---|---|---|---|---|---|---|
| MCF 7 | 13.39 | 31.017 4789 | 4.93854 E-06 | 25.5072 5555 | 0.00022 5082 | 31.606 663 | 3.28274 E-06 |
| MCF 10A | 13.17571 | 29.802 2518 | 9.88356 E-06 | 24.9255 2948 | 0.00029 0371 | NE* | |
| MDA | 12.25686 | 23.602 5486 | 0.00038 4245 | 19.5566 9403 | 0.00634 6472 | 25.994 686 | 7.31987 E-05 |

| | | | | | | | |
|---|---|---|---|---|---|---|---|
| *NE: No expression. | | | | | | | |

**Table 14 - Raw Cq values and expression of the reference and target genes in peripheral blood samples from healthy donors (D).**

| **Donors** | **RPL13a Cq** | **GLUT1 Cq** | **GLUT1 2^-(ΔCq)** | **HIF 1a Cq** | **HIF 1a 2^-(ΔCq)** | **CAIX Cq** | **CAIX 2^-(ΔCq)** |
|---|---|---|---|---|---|---|---|
| **D1** | 13.681624 | 26.10739 | 0.000181749 | 23.46668 | 0.001133454 | NE* | |
| **D2** | 12.465688 | 26.50502 | 5.93935E-05 | 20.41321 | 0.004050966 | 36.42058 | 6.1498E-08 |
| **D3** | 14.691833 | 27.76325 | 0.000116175 | 23.84112 | 0.001761126 | NE* | |
| **D4** | 15.800835 | 30.21148 | 4.59161E-05 | 26.06954 | 0.000810611 | 37.30807 | 3.3549E-07 |
| **D5** | 18.286335 | 33.20669 | 3.22497E-05 | 30.3742 | 0.000229716 | NE* | |
| **D6** | 10.767191 | 27.03734 | 1.26531E-05 | 21.40056 | 0.000629558 | 35.75698 | 3.0014E-08 |
| **D7** | 12.149372 | 26.92645 | 3.5617E-05 | 22.7225 | 0.000656403 | 36.23159 | 5.6303E-08 |
| **D8** | 17.060295 | NE* | | 28.31106 | 0.000410376 | NE* | |
| **D9** | 16.459913 | 29.41041 | 0.000126332 | 26.88411 | 0.000727787 | NE* | |
| **D10** | 17.508467 | NE* | | 28.05116 | 0.000670401 | NE* | |
| **D11** | 16.30954 | 31.3649 | 2.93687E-05 | 30.33558 | 5.99434E-05 | NE* | |
| **D14** | 22.516657 | 39.58594 | 7.27168E-06 | 30.05666 | 0.005373214 | NE* | |
| **D15** | 16.880482 | 29.50766 | 0.000158067 | 24.56055 | 0.004876065 | NE* | |
| **D16** | 17.75515 | 31.85599 | 5.69148E-05 | 25.87629 | 0.00359164 | NE* | |
| **D17** | 16.567553 | 30.06822 | 8.62769E-05 | 24.92467 | 0.003049705 | NE* | |
| **D19** | 17.795258 | 32.34932 | 4.15711E-05 | 26.71941 | 0.002058555 | NE* | |
| **D20** | 22.993053 | 40.26694 | 6.31016E-06 | 33.06282 | 0.000930461 | NE* | |
| **D21** | 16.647888 | 31.05007 | 4.61862E-05 | 26.02197 | 0.001507019 | NE* | |
| **D22** | 17.917637 | 35.15688 | 6.46355E-06 | 26.62634 | 0.002390119 | NE* | |
| **D23** | 21.146254 | NE* | | 30.07137 | 0.002057173 | NE* | |
| **D24** | 16.726357 | 30.99241 | 5.07564E-05 | 25.58646 | 0.002152001 | NE* | |
| **D25** | 23.975517 | NE* | | 31.89375 | 0.00413404 | NE* | |
| **D26** | 13.799793 | 27.5015 | 7.50542E-05 | 22.70199 | 0.002090125 | 38.05107 | 5.0077E-08 |
| **D27** | 14.928394 | 28.25807 | 9.71332E-05 | 23.11997 | 0.003420512 | 37.4409 | 1.6713E-07 |
| **D28** | 26.411465 | NE* | | 34.28094 | 0.004276125 | NE* | |

| | | | | | | | |
|---|---|---|---|---|---|---|---|
| *NE: No expression. | | | | | | | |

dPCR analyses for the tested genes provide more accurate, reproducible, and statistically significant results.

### Example 5 - Assessment of the diagnostic and/or prognostic potential of LIF

To assess the potential of *LIF* as a diagnostic and prognostic marker in liquid biopsy, the association of *LIF* gene expression with *HIF-1α* and *HPSE* in breast cancer patients was investigated, as well as its association with *Oncotype* values and clinical data (staging, progression, time to progression, age, and presence of estrogen and progesterone receptors).

To evaluate *LIF* expression using real-time PCR (qPCR) and its association with clinicopathological variables, blood samples from breast cancer patients were analyzed. Women over 18 years of age with a confirmed diagnosis of breast cancer based on clinicopathological tests, and with no history of diabetes, kidney disease, or cardiovascular disease, were included. Patients who did not meet this inclusion criterion or who refused to participate in the study were excluded. Peripheral blood samples were obtained and analyzed from these patients at the time of diagnosis and 3 and 6 months after the start of chemotherapy treatment. As controls, peripheral blood samples were used from healthy women (donors), matched for age, with no history of breast cancer (confirmed by annual routine tests and breast cancer screening) and no previous kidney, cardiovascular disease, or diabetes. In total, samples from 121 breast cancer patients and from 80 healthy donors were analyzed. The donors were matched by age, with a mean age of 44.15 years (44.15 ± 7), considering the mean age of the cancer patients 52.7 years (52.7 ± 11).

The clinical characteristics of these patients and of healthy women were recorded (Table 15).

**Table 15 - Clinical characteristics of the patients included in the study.**

| **Clinical Characteristics** | **N** | **%** |
|---|---|---|
| **Clinical Stage** | | |
| 0/I | 33 | 27.3 |
| II | 59 | 48.8 |
| III | 29 | 23.9 |

| **Progression** | | |
|---|---|---|
| Negative | 110 | 92.4 |
| Positive | 9 | 7.6 |

| **Estrogen Receptor** | | |
|---|---|---|
| Negative | 30 | 25.2 |
| Positive | 89 | 74.8 |

| **Progesterone Receptor** | | |
|---|---|---|
| Negative | 50 | 42.0 |
| Positive | 69 | 58.0 |

| **Her_ihc*** | | |
|---|---|---|
| Negative | 28 | 23.7 |
| +/3+ | 36 | 30.5 |
| ++/3+ | 23 | 19.5 |
| +++/3+ | 31 | 26.3 |

| | **Median (p.25; p.75)** | **Min.; Max.** |
|---|---|---|
| Age | 53 (47.5; 61.5) | 27; 78 |
| Follow-up time (months) | 28 (25; 32) | 4; 49 |

| | | |
|---|---|---|
| p.25; p.75: 25th-75th percentiles, respectively; Min.: Minimum; Maximum: Maximum; * Immunohistochemical classification of Her2 tumor status according to the good pathology practices; 3+ = Her2 positive tumors. | | |

For qPCR, total RNA was extracted from 5 mL peripheral blood samples from patients and donors using the TRIzol reagent (Invitrogen, Carlsbad, CA, USA, cat. no. 15596026) following the manufacturer's recommendations. Subsequently, these RNA samples (initial 1 µg) were converted to cDNA using the QuantiTect Reverse Transcription kit (Qiagen, cat no 205311), according to the manufacturer's protocol.

Specific primers for LIF were designed using Primer3 Input 0.4.0 software, available at //frodo.wi.mit.edu/primer3/. The primer sequences thus designed were checked for specificity using the Primer-BLAST program, available at //www.ncbi.nlm.nih.gov/tools/primer-blast. To normalize the relative expression of the target gene, mean expression values of the RPL13α gene, a ribosomal protein that is part of the 60S subunit composition, were used. The sequences of the designed primers and the characteristics of their amplicons are described in Table 16.

**Table 16 - Characteristics of specific primers and their amplicons.**

| **GENE** | **SEQUENCE (5'-3')** | **AMPLICON (bp)** |
|---|---|---|
| *LIF* | F- SEQ ID NO: 18 | 124 |
| | R- SEQ ID NO: 19 | |
| *RPL13a* | F- SEQ ID NO: 16 | 126 |
| | R- SEQ ID NO: 17 | |
| *HIF-1α* | F- SEQ ID NO: 1 | 227 |
| | R- SEQ ID NO: 2 | |

The initial standardization of qPCR amplifications was performed in an Applied Biosystems 7500 Real Time PCR Systems thermocycler (Applied Biosystems, Foster City, CA, USA) in a final volume of 15 µL containing: 1X SYBR Green mix (Quantitec SYBR Green PCR kit, QIAGEN cat. no. 204054), 10 pmol of each specific primer and 2 µL of cDNA (initially diluted 10X). The initial parameters of the cycle consisted of an initial hot start stage at 95°C for 10 min, followed by 40 cycles of 95°C for 15 seconds, and 60°C for 25 seconds. The calibration curve for the gene under study was made with serial dilutions of cDNA synthesized from 1 µg of mRNA from the MCF7 (tumorigenic) cell line. The difference in *LIF* expression in the included samples was determined by formula (I). ${2}^{∧} \left(-ΔCq\right)$

Initially, a descriptive analysis was performed in which the defined qualitative variables were expressed in absolute and relative frequencies, while the quantitative variables were summarized in means, standard deviations, minimum and maximum values. In general, the Mann-Whitney test was used for non-parametric data and the Student's t-test for parametric data. Regarding data statistical analysis for more than 2 selected groups, the Kruskal-Wallis test was used for non-parametric data and one-way ANOVA for parametric data. In data correlation analysis, Spearman's test was used for non-parametric data and Pearson's test for parametric data.

To evaluate the expression of the proposed gene in breast cancer patients and the control group, the Student's t-test was used. Furthermore, repeated measures analysis of variance (ANOVA) was used to compare the evolution of the proposed gene expression over time and according to some covariates (staging, progression, progression time, age, presence of estrogen and progesterone receptors, and molecular expression of *HIF-1α,* heparanase, and *Oncotype*). A 95% confidence interval was used for all analyses. Statistical calculations were carried out using NCSS and GraphPad Prism software.

### Results

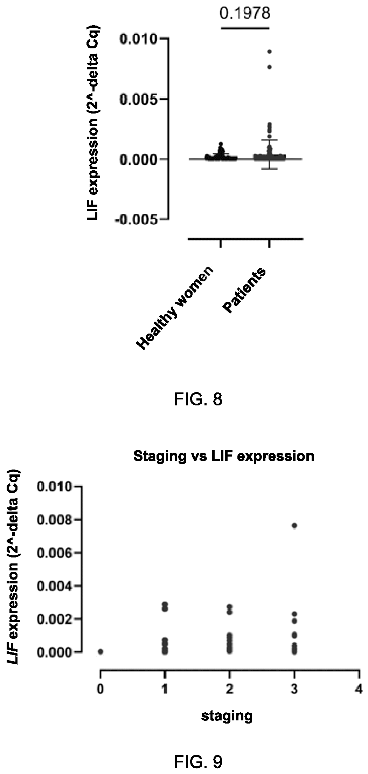

In order to evaluate the applicability of using LIF expression as a diagnostic marker, a comparison was made between the mean expression of this gene in peripheral blood of healthy donors and breast cancer patients at the time of diagnosis (Fig. 8). As observed, there was no correlation between the difference in expression between patients and donors (p = 0.1978), indicating that there is an increase in *LIF* expression in only some patients.

In order to verify if there is a relationship between *LIF* expression and breast cancer stage at the time of diagnosis, potentially extending to a possible complementary use as a prognostic marker, a comparison was made between the expression of this gene and the neoplasm stage. However, there was no statistically significant difference between the stages, with LIF expression being uniformly distributed across the stages (Fig. 9).

In healthy mammary epithelial cells, the *LIF* promoter is hypermethylated, but extensively demethylated during breast cancer progression, so its overexpression is commonly associated with worse recurrence-free survival in breast cancer patients. Surprisingly, the results shown herein demonstrate that LIF expression in peripheral blood of breast cancer patients does not differ from that found in healthy women, ruling out this marker as an auxiliary tool in the diagnosis of this disease through liquid biopsy.

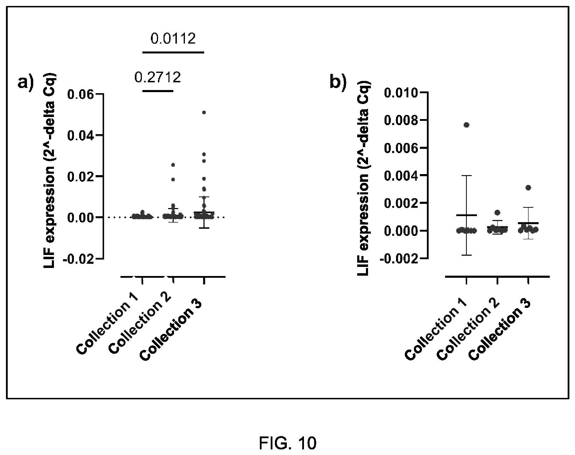

To evaluate the potential use of *LIF* as a marker of therapeutic success and prognosis, comparisons were made between the mean expressions of this gene in serial peripheral blood collections during treatment, wherein collection 1 refers to the sample collected at the time of diagnosis, collection 2 was performed 3 months after chemotherapy treatment, and collection 3 was performed 6 months after the start of treatment. In this analysis, patients were divided according to their progression, with positive cases indicating an increase in tumor stage and negative cases indicating that the cancer regressed or did not progress (Fig. 10, items A and B).

It can be observed that there was an increase in *LIF* expression between collections 1 and 3 in patients who had a negative disease progression with treatment, that is, there was an increase in *LIF* in the blood of patients who responded to treatment during their follow-up. This finding was not observed in those patients who experienced breast cancer progression, either with stage worsening or the appearance of metastases, indicating that the response to treatment was insufficient to at least stabilize the disease.

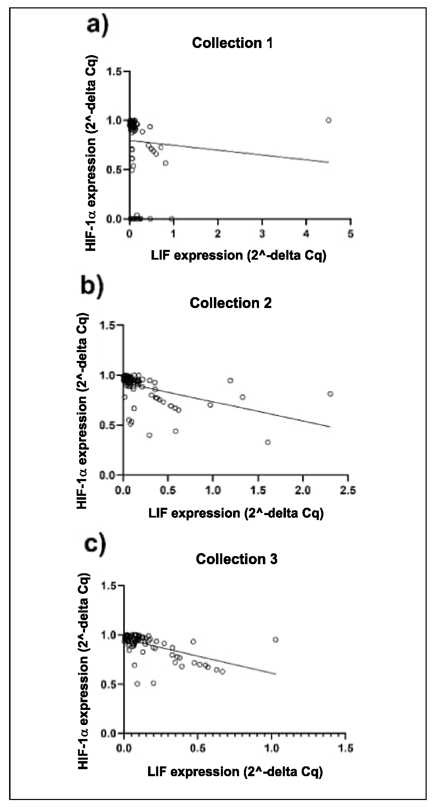

Next, a correlation was performed between the expression of *LIF* and *HIF-1α,* whose values were obtained by Peiró et al., 2021 and were found to be higher in breast cancer patients at the time of diagnosis, regardless of the stage. This analysis was performed with the aim of complementing the results for greater applicability in clinical practice, since the expression of *HIF-1α* in the blood has been seen as a diagnostic marker. The correlation between *LIF* and *HIF-1α* expression at different blood collection times was negative and moderate (Fig. 11, items A, B, and C).

Overexpression of *LIF* analyzed in tumor samples is commonly associated with poor prognosis in breast cancer patients ⁶¹. An assessment of *LIF* expression in malignant or non-malignant human breast cancer cell lines, such as *T47D, MCF7, SK-Br-3, HS578T, MDA-MB-232, BT474* and *MDA-MB-*468, showed a correlation between LIF expression levels and metastatic capacity of cell lines, with higher levels in cells having a greater metastatic capacity ⁵¹. Surprisingly, the results shown herein demonstrate that in peripheral blood the *LIF* gene has a protective role against uncontrolled proliferation or tumor suppression, especially considering its correlation with *HIF-1α* expression in peripheral blood of such patients: it is known that increased expression of hypoxia-inducible factors, such as *HIF-1α,* confers greater aggressiveness, invasion and metastatic tumor capacity, worse prognosis, decreased survival, increased recurrence and therapeutic resistance due to alterations in cellular metabolism ^{62,17,20,63}. In this experiment, it was observed that *LIF* expression is negatively correlated with *HIF-1α* in peripheral blood samples from breast cancer patients, such that an increase in *HIF-1α* expression is associated with lower *LIF* expression, a result that demonstrates its prognostic potential.

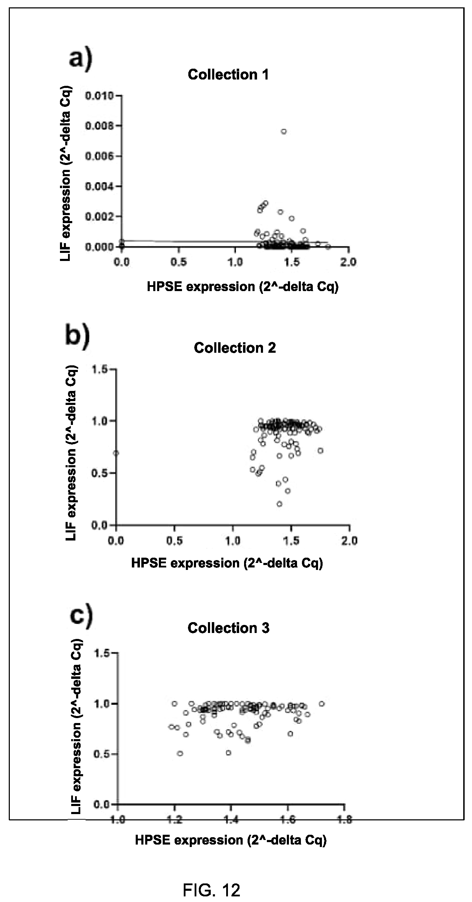

In order to verify whether it is possible to analyze the local behavior of the tumor through the expression of *LIF* in blood, a correlation was made between its expression and that of heparanase at the time of diagnosis of the neoplasm. As a result, there was a moderate negative correlation between heparanase and *LIF* expression (Fig. 12 item A; p = 0.0006 and r = - 0.3180). In subsequent collections (carried out after the start of chemotherapy treatment) it was not possible to establish a correlation between these two markers (Fig. 12, items B and C).

As mentioned earlier, heparanase is an enzyme associated with cancer progression that promotes tumor growth, angiogenesis, and metastasis ⁶⁴. The expression of heparanase by different cell types within the tumor microenvironment contributes to aggressive tumor behavior through the upregulation of protease expression and activity ⁶⁵. Thus, increased heparanase expression has been correlated with an unfavorable prognosis in cancer patients due to its involvement in angiogenesis and tumor progression ⁶⁴. Surprisingly, based on the results obtained herein, the expression of *LIF* and heparanase in peripheral blood of breast cancer patients is inversely proportional, such that an increase in *LIF,* with its suppressive activity, is accompanied by a reduction in heparanase expression. This interaction reflects the response to ongoing chemotherapy treatment and reinforces the prognostic potential of *LIF.*

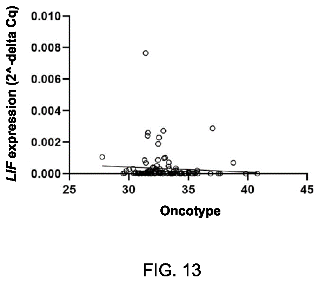

Finally, the correlation between the expression of LIF, a widely applicable, fast, easily accessible, and low-cost test, and the *Oncotype* test of each patient was evaluated (which is a genetic test performed based on the expression of 21 genes in tumor tissue to indicate therapeutic response and the probability of disease recurrence). The importance of Oncotype DX^{®} in guiding treatment decisions is highlighted by Biroschak et al. ⁶⁶, who emphasize that the test provides risk stratification based on the recurrence score, associated with distant recurrence and response to chemotherapy. Since *Oncotype* is expensive and difficult to access, a prognostic platform with the same biomarkers covered by the *Oncotype* ⁶⁷ test was established and validated. From this assessment, a surprisingly moderate negative correlation was found (Fig. 13, p = 0.0186 and r = -0.2201) between *LIF* expression in peripheral blood of breast cancer patients and the score generated by the prognostic platform in tissue samples from these women, such that the higher the multigene score, the worse the prognosis, and the lower the *LIF* expression in peripheral blood.

Although increased *LIF* expression in tumor cells is an indicator of a worse prognosis, in peripheral blood it represents a marker of a good prognosis. Based on the data disclosed here, it was observed that *LIF* expression increases during treatment in patients who experienced negative progression, i.e., responded to treatment, indicating that this gene can be used as a prognostic marker. A negative correlation was also observed in peripheral blood of breast cancer patients with the expression levels of HIF1α and heparanase - markers already established as indicators of poor prognosis - and with the *Oncotype* scores obtained from tumor samples from these patients. These data reinforce its applicability as a prognostic marker.

dPCR analyses for the tested genes provide more accurate, reproducible, and statistically significant results.

Based on the results shown, it can be concluded that the proposed *in vitro* process is able to detect the expression of hypoxia-inducible factor (*HIF-1a*) in liquid biopsy samples, as well as the increase in its expression in peripheral blood of women with breast cancer, compared to healthy women. This detection of differential *HIF-1α* expression allows the use of the present invention in the diagnosis of breast cancer. Furthermore, by analyzing total RNA samples, the present invention has broad potential for use in breast cancer screening programs and can be used to indicate the presence of the disease before it is detected by routine imaging tests.

The quantification of *HIF-1α* expression can be performed using both qPCR and dPCR. The present *in vitro* process is an alternative to the prior art breast cancer diagnostic methods and uses gene expression quantification by dPCR to obtain more accurate, reproducible, and statistically significant results.

Finally, the expression of other genes of interest can also be quantified in order to complement the present invention. Based on the results shown herein, it can be concluded that the present *in vitro* process can additionally include the quantification of gene expression of *MCT1, CD147,* and *GLUT1* to increase the accuracy of results related to the diagnosis of breast cancer. Further quantification of *MCT4* and *LIF* gene expression imparts the process with prognostic utility. Thus, the present invention provides an *in vitro* process for tumor diagnosis and/or prognosis, a kit, as well as an *in vitro* process for monitoring the clinical status of an individual with breast cancer under treatment.

Those skilled in the art will appreciate the knowledge shown herein and will be able to reproduce the invention in the forms shown and in other variants and alternatives, covered by the scope of the following claims.

### References

1) INCA - Instituto Nacional de Câncer. Síntese de Resultados e Comentários. Câncer de mama. https://www.gov.br/inca/pt-br/assuntos/cancer/numeros/estimativa/sintese-de-resultados -e-comentarios. Acesso em 20 de abril de 2023.
2) Sznurkowska MK, Aceto N. The gate to metastasis: key players in cancer cell intravasation. FEBS J. 2022; 289(15): 4336-4354.
3) Weigelt B, Peterse JL, van't Veer LJ. Breast cancer metastasis: markers and models. Nature Reviews 2005; 5: 591-602
4) Curry JM, Tuluc M, Whitaker-Menezes D, Ames JA, Anantharaman A, Butera A, Leiby B, Cognetti DM, Sotgia F, Lisanti MP, Martinez-Outschoorn UE. Cancer metabolism, stemness and tumor recurrence. Cell Cycle 2013; 12: 1371-1384.
5) Thorne JL, Campbell MJ. Nuclear receptors and the Warburg effect in cancer. Int J Cancer 2015; 137: 1519-1527.
6) Folkman J. What is the evidence that tumors are angiogenesis dependent?. Journal of the National Cancer Institute, 1990; 82(1): 4-6.
7) Badowska-Kozakiewicz AM, Budzik MP, Przybylski J. Hypoxia in breast cancer. Pol J Pathol 2015; 66: 337-346.
8) Devic S. Warburg effect - a consequence or the cause of carcinogenesis? J Cancer 2016; 7: 817-822.
9) Vander Heiden MG, Cantley LC, Thompson CB. Understanding the Warburg effect: the metabolic requirements of cell proliferation. Science. 2009; 324(5930): 1029-33.
10) Calcinotto A, Filipazzi P, Grioni M, lero M, De Milito A, Ricupito A, Cova A, Canese R, Jachetti E, Rossetti M, Huber V, Parmiani G, Generoso L, Santinami M, Borghi M, Fais S, Bellone M, Rivoltin L. Modulation of microenvironment acidity reverses anergy in human and murine tumor-infiltrating T lymphocytes. Cancer Res 2012; 72: 2746-2756.
11) Vazquez A, Liu J, Zhou Y, Oltvai ZN. Catabolic efficiency of aerobic glycolysis: The Warburg effect revisited. BMC Systems Biology 2010; 4: 58.
12) Wang GL, Jiang BH, Rue EA, Semenza GL. Hypoxia-inducible factor 1 is a basic-helix-loop-helix-PAS heterodimer regulated by cellular O2 tension. Proc. Natl. Acad. Sci. USA 1995; 92: 5510-5514.
13) Jaakkola P, Mole DR, Tian YM, Wilson MI, Gielbert J, Gaskell SJ, von Kriegsheim A, Hebestreit HF, Mukherji M, Schofield CJ, Maxwell PH, Pugh CW, Ratcliffe PJ. Targeting of HIF alpha to the von Hippel-Lindau ubiquitylation complex by O2-regulated prolyl hydroxylation. Science 2001; 292: 468-472.
17) Semenza GL. HIF-1: mediator of physiological and pathophysiological responses to hypoxia, J. Appl. Physioll. 2000; 88: 1474-1480.
18) Marín-Hernandez A, Gallardo-Pérez JC, Ralph SJ, Rodriguez-Enríquez S, Moreno-Sánchez R. HIF-1alpha modulates energy metabolism in cancer cells by inducing over-expression of specific glycolytic isoform. Mini Rev. Med. Chem. 2009; 9: 1084-1101.
19) Rohwer N, Cramer T. Hypoxia-mediated drug resistance: novel insights on the functional interactions of HIFs and cell death pathways. Drug Resist. Updat. 2011; 14: 191-201.
20) Lara PC, Lloret M, Clavo B, Apolinario RM, Henríquez-Hernández LA, Bordón E, Fontes F, Rey A. Severe hypoxia induces chemo-resistance in clinical cervical tumors through MVP over-expression. Radiat. Oncoll. 2009; 4: 20.
21) Rankin EB, Giaccia AJ. Hypoxic control of metastasis. Science 2016; 352(6282): 175-180.
22) He G, Jiang Y, Zhang B, Wu G. Th effect of HIF-1a on glucose metabolism, growth and apoptosis of pancreatic cancerous cells. Asia Pac. J. Clin. Nutr. 2014; 23: 174-180.
23) Bos R, Zhong H, Hanrahan CF, Mommers ECM, Semenza GL, Pinedo HM, Abeloff MD, Simons JW, Diest PJ, van der Wall E. Levels of Hypoxia-Inducible Factor-1_ During Breast Carcinogenesis. J. Natl Cancer Inst. 2001; 93: 309-314.
24) INCA- Instituto Nacional de Cancer. Cancer de mama: vamos falar sobre isso? Disponível em: https://www.inca.gov.br/sites/ufu.sti.inca.local/files/media/document/cartil1.pdf. Acesso em 21 de abril 2023.
25) Pashayan N, Antoniou AC, Ivanus U, Esserman LJ, Easton DF, French D, Sroczynski G, Hall P, Cuzick J, Evans DG, Simard J, Garcia-Closas M, Schmutzler R, Wegwarth O, Pharoah P, Moorthie S, De Montgolfier S, Baron C, Herceg Z, Turnbull C, Balleyguier C, Rossi PG, Wesseling J, Ritchie D, Tischkowitz M, Broeders M, Reisel D, Metspalu A, Callender T, de Koning H, Devilee P, Delaloge S, Schmidt MK, Widschwendter M. Personalized early detection and prevention of breast cancer: ENVISION consensus statement. Nat Rev Clin Oncoll. 2020; 17(11): 687-705
26) Ministério da Saúde. Exames de investigação diagnóstica para câncer de mama podem ser feitos pelo SUS. Disponível em: https://www.gov.br/saude/pt-br/assuntos/noticias/2022/outubro/exames-de-investigacaodiagnostica-para-cancer-de-mama-podem-ser-feitos-pelo-sus#:~:text=Segundo%20reco menda%C3%A7%C3%B5es%20atuais%20do%20Minist%C3%A9rio,seja%2C %20ant es%20que%20seja%20palp%C3%A1vel. Acesso em 21 de abril 2023.
27) Sociedade Brasileira de Mastologia (SBM). Incidência de mulheres com cancer de mama e menos de 35 anos está entre 4% e 5%. Disponivel em: https://www.sbmastologia.com.br/incidencia-de-mulheres-com-cancer-de-mama-com-m enos-de-35-anos-esta-entre-4-e-5-dos-casos/. Acesso em 21 de abril 2023. Waks AG, Kim D, Jain E, Snow C, Kirkner GJ, Rosenberg SM, Oh C, Poorvu PD, Ruddy KJ, Tamimi RM, Peppercorn J, Schapira L, Borges VF, Come SE, Brachtel EF, Warner E, Collins LC, Partridge AH, Wagle N. Somatic and Germline Genomic Alterations in Very Young Women with Breast Cancer. Clin Cancer Res. 2022 Jun 1;28(11):2339-2348.
28) Franzoi M A, Rosa D D, Zaffaroni F, Werutsky G, Simon S, Bines J, Barrios C, Cronemberger E, Queiroz GS, Cordeiro de Lima V, Júnior RF, Couto J, Emerenciano K, Resende H, Crocamo S, Reinert T, Van Eyli B, Nerón Y, Dybal V, Lazaretti N, ... Liedke PER. Advanced Stage at Diagnosis and Worse Clinicopathologic Features in Young Women with Breast Cancer in Brazil: A Subanalysis of the AMAZONA III Study (GBECAM 0115). Journal of Global Oncology 2019; 5: 1-10.
29) Waks AG, Kim D, Jain E, Snow C, Kirkner GJ, Rosenberg SM, Oh C, Poorvu PD, Ruddy KJ, Tamimi RM, Peppercorn J, Schapira L, Borges VF, Come SE; Brachtel EF, Warner E, Collins LC, Partridge AH, Wagle N. Somatic and Germline Genomic Alterations in Very Young Women with Breast Cancer. Clin Cancer Res 2022; 28(11): 2339-2348.
30) Rodriguez J, Avila J, Rolfo C, Ruíz-Patiño A, Russo A, Ricaurte L, Ordóñez-Reyes C, Arrieta O, Zatarain-Barrón ZL, Recondo G, Cardona AF. When tissue is an issue the liquid biopsy is nonissue: a review. Oncol Ther. 2021; 9: 89-110.
32) Caputo V, Ciardiello F, Della Corte CM, Martini G, Troiani T, Napolitano S. Diagnostic value of liquid biopsy in the era of precision medicine: 10 years of clinical evidence in cancer. Explor Target Antitumor Ther. 2023; 4: 102-38
33) Diel IJ, Cote RJ. Bone marrow and lymph node assessment for minimal residual disease in patients with breast cancer. Cancer Treat Rev 2000; 26: 53-65.
34) Ring A, Smith IE, Dowsett M. Circulating tumour cells in breast cancer. Lancet Oncol 2004; 5: 79-88.
35) Ma M, Zhu H, Zhang C, Sun X, Gao X, Chen G. "Liquid biopsy" - ctDNA detection with great potencial and challenges. Annals of Translational Medicine 2015; 3: 235-241.
36) Apostolaki S, Perraki M, Pallis A, Bozionelou V, Agelaki S, Kanellou P, Kotsakis A, Politaki E, Kalbakis K, Kalykaki A, Vamvakas L, Georgoulias V, Mavroudis D. Circulating HER2 mRNA-positive cells in the peripheral blood of patients with stage I and II breast cancer after the administration of adjuvant chemotherapy: evaluation of their clinical relevance. Ann Oncoll. 2007; 18: 851-8. Erratum in: Ann Oncoll. 2007; 18(11): 1916.
37) Ignatiadis M, Perraki M, Apostolaki S, Politaki E, Xenidis N, Kafousi M, Stathopoulos E, Lianidou E, Sotiriou C, Georgoulias V, Mavroudis D. Molecular detection and prognostic value of circulating cytokeratin-19 messenger RNA-positive and HER2 messenger RNA-positive cells in the peripheral blood of women with early-stage breast cancer. Clin Breast Cancer 2007; 7: 883-889.
38) Strati A, Markou A, Parisi C, Politaki E, Mavroudis D, Georgoulias V and Lianidou E. Gene expression profile of circulating tumor cells in breast cancer by RT-qPCR. BMC Cancer 2011; 11: 422
39) Peiró CHF, Perez MM, de Aquino GSA, Encinas JFA, Sousa LVA, da Veiga GL, Del Giglio A, Fonseca FLA, da Costa Aguiar Alves B. Diagnostic potential of hypoxia-induced genes in liquid biopsies of breast cancer patients. Sci Rep. 2021; 11(1): 8724.
42) Corcoran SE, O'Neill LA. HIF1α and metabolic reprogramming in inflammation. J Clin Invest. 2016; 126(10): 3699-3707.
43) Bollinger T, Bollinger A, Gies S, Feldhoff L, Solbach W, Rupp J. Transcription regulates HIF-1α expression in CD4(+) T cells. Immunol Cell Bioll. 2016; 94(1): 109-113.
44) Taylor SC, Laperriere G, Germain H. Droplet Digital PCR versus qPCR for gene expression analysis with low abundant targets: from variable nonsense to publication quality data. Sci Rep 2017; 7: 2409.
45) Falzone L, Musso N, Gattuso G, Bongiorno D, Palermo Cl, Scalia G, Libra M, Stefani S. Sensitivity assessment of droplet digital PCR for SARS-CoV-2 detection. Int J Mol Med 2020; 46: 957-964.
46) Hindson CM, Chevillet JR, Briggs HA, Gallichotte EN, Ruf IK, Hindson BJ, Vessella RL, Tewari M. Absolute quantification by droplet digital PCR versus analog real-time PCR. Nat Methods 2013; 10: 1003-1005.
47) Gutiérrez-Aguirre I, Rački N, Dreo T, Ravnikar M. (2015) Droplet Digital PCR for Absolute Quantification of Pathogens. In: Lacomme C. (eds) Plant Pathology. Methods in Molecular Biology, vol 1302. Humana Press, New York, NY
48) Taylor SC, Carbonneau J, Shelton DN, Boivin G. Optimization of Droplet Digital PCR from RNA and DNA extracts with direct comparison to RT-qPCR: Clinical implications for quantification of Oseltamivir-resistant subpopulations. J Virol Methods 2015; 224: 58-66.
49) Kuphal S, Wallner S, Bosserhoff AK. Impact of LIF (leukemia inhibitory factor) expression in malignant melanoma. Exp Mol Patholl. 2013;95(2):156-65.
50) Fan YY, Zhang JM, Wang H, Liu XY, Yang FH. Leukemia inhibitory factor inhibits the proliferation of primary rat astrocytes induced by oxygen-glucose deprivation. Acta Neurobiol Exp. 2013;73(4):485-494.
51) Vaziri N, Shariati L, Javanmard SH. Leukemia inhibitory factor: a main controller of breast cancer. J Biosci. 2020;45:143.
52) Liu B, Lu Y, Li J, Liu Y, Liu J, Wang W. Leukemia inhibitory factor promotes tumor growth and metastasis in human osteosarcoma via activating STAT3. APMIS. 2015;123(10):837-46.
53) Jiang W, Bai W, Li J, Liu J, Zhao K, Ren L. Leukemia inhibitory factor is a novel biomarker to predict lymph node and distant metastasis in pancreatic cancer. Int J Cancer 2021;148(4):1006-1013.
54) Halder S, Parte S, Kshirsagar P, Muniyan S, Nair HB, Batra SK, Seshacharyulu P. The pleiotropic role, functions and targeted therapies of LIF/LIFR axis in cancer: Old spectacles with new insights. Biochim Biophys Acta Rev Cancer. 2022;1877(4):188737.
55) Yersal O, Barutca S. Biological subtypes of breast cancer: Prognostic and therapeutic implications. World J Clin Oncoll. 2014;5(3):412-424.
56) Paik S, Shak S, Tang G, Kim C, Baker J, Cronin M, Baehner FL, Walker MG, Watson D, Park T, Hiller W, Fisher ER, Wickerham DL, Bryant J, Wolmark N. A multigene assay to predict recurrence of tamoxifen-treated, node-negative breast cancer. N Engl J Med. 2004;351:2817-2826.
57) Habel LA, Quesenberry CP, Jacobs M, Greenberg D, Fehrenbacher L, Alexander C, Baker J, Watson D, Hackett J, Shak S. Gene expression and breast cancer mortality in Northern California Kaiser Permanente patients: A large population-based case control study. J Clin Oncol 2005;23(suppl 18S):603.
58) Jayatilleke KM, Hulett MD. Heparanase and the hallmarks of cancer. J Transl Med. 2020;18(1):453.
59) Baraz L, Haupt Y, Elkin M, Peretz T, Vlodavsky I. Tumor suppressor p53 regulates heparanase gene expression. Oncogene. 2006;25(28):3939-3947.
60) Christianson J, Oxford JT and Jorcyk CL. Emerging Perspectives on Leukemia Inhibitory Factor and its Receptor in Cancer. Front. Oncoll. 2021;11:693724.
61) Yue X, Wang J, Chang C, Liu J, Yang X, Zhou F, Qiu X, Bhatt V, Guo JY, Su X, Zhang L, Feng Z, Hu W. Leukemia inhibitory factor drives glucose metabolic reprogramming to promote breast tumorigenesis. Cell Death &Amp; Disease 2022;13(4):370.
62) Fallah J, Rini BI. HIF Inhibitors: status of current clinical development. Curr Oncol Rep. 2019;21(1):6.
63) Ke Q, Costa M. Hypoxia-inducible factor-1 (HIF-1). Mol Pharmacoll. 2006; 70(5):1469-80.
64) Ostapoff, K. T., Awasthi, N., Cenik, B. K., Hinz, S., Dredge, K., Schwarz, R. E., ... & Brekken, R. A. (2013). Pg545, an angiogenesis and heparanase inhibitor, reduces primary tumor growth and metastasis in experimental pancreatic cancer. Molecular Cancer Therapeutics, 12(7), 1190-1201.
65) Purushothaman, A., Chen, L., Yang, Y., & Sanderson, R. D. (2008). Heparanase stimulation of protease expression implicates it as a master regulator of the aggressive tumor phenotype in myeloma. Journal of Biological Chemistry, 283(47), 32628-32636.
66) Biroschak JR, Schwartz GF, Palazzo JP, Toll AD, Brill KL, Jaslow RJ, Lee SY. Impact of Oncotype DX on treatment decisions in ER-positive, node-negative breast cancer with histologic correlation. Breast J. 2013 May-Jun;19(3):269-75.
67) Kuniyoshi RK, Gehrke Fde S, Alves BC, Vilas-Bôas V, Coló AE, Sousa N, Nunes J, Fonseca FL, Del Giglio A. Gene profiling and circulating tumor cells as biomarker to prognostic of patients with locoregional breast cancer. Tumour Bioll. 2015 Sep;36(10):8075-83.

## Claims

1. An *in vitro* process for tumor screening, diagnosis and/or prognosis **characterized in that** it comprises the steps of:
a) preparing an analyte from a liquid sample;
b) quantifying the expression of *HIF-1α* from the total RNA in the sample;
c) optionally, normalizing the quantification of *HIF-1α* expression from the quantification of a reference marker; and
d) comparing the quantification of the *HIF-1α* expression to a predetermined level, indicating the presence of a tumor when the value is above said level, or the absence of a tumor when the value is below said level.

2. The *in vitro* process of claim 1 **characterized in that** it additionally comprises a step of expression quantification of one or more genes among: *GLUT1*; monocarboxylate transporters *MCT*; MCT chaperone *CD147*; Leukemia Inhibitory Factor *LIF.*

3. The *in vitro* process of any one of claims 1-2 **characterized in that** the liquid sample is blood, and the quantification of total RNA is performed by dPCR.

4. The *in vitro* process of any one of claims 1-3 **characterized in that** step b) comprises the synthesis of cDNA from 100 ng of total RNA and the dilution of the synthesized cDNA 25 times or 50 times.

5. The *in vitro* process of any one of claims 1-4 **characterized in that** step b) further comprises the amplification of *HIF-1α* cDNA, wherein the primers/probes for *HIF-1α* cDNA amplification are selected from the nucleotide sequences of: Seq ID No:1; Seq ID No: 2; Seq ID No: 3, or combinations thereof.

6. The *in vitro* process of any one of claims 1-5 **characterized in that** said normalization step is performed by comparison with the expression level of *RPL13a, GUSB, TRFC,* and/or *Beta-actin.*

7. The *in vitro* process of any one of claims 1-6 **characterized in that** the comparison step d) is performed by analyzing the Receptor Operating Characteristic (ROC) curve.

8. The *in vitro* process of any one of claims 1-7 **characterized in that** *HIF-1α* expression values above 5,010 transcript copies/uL indicate the presence of a tumor, and values below 5,010 transcript copies/µL indicate the absence of a tumor.

9. The *in vitro* process of any one of claims 1-8 **characterized in that** the tumor is breast cancer.

10. A kit for screening, diagnosis and/or prognosis of tumors, **characterized in that** it comprises:
- reagents for stabilizing the total RNA in the liquid sample;
- enzyme to enable the formation of cDNA from the mRNA of the *HIF-1α* gene and, optionally, from the mRNA of a reference marker gene;
- primers, probes, dNTPs for amplification of *HIF-1α* cDNA and, optionally, of the reference marker gene cDNA;
- reagents for the quantitative visualization of amplified cDNAs.

11. The kit of claim 10 **characterized in that** the primers/probes for *HIF-1α* cDNA amplification are selected from the following nucleotide sequences: Seq ID No:1; Seq ID No: 2; Seq ID No: 3, or combinations thereof.

12. The kit of any one of claims 10-11 **characterized in that** it additionally comprises primers and probes for the amplification of cDNA of one or more genes from among: *GLUT1*; monocarboxylate transporters *MCT*; MCT chaperone *CD147*; Leukemia Inhibitory Factor *LIF.*

13. The kit of any one of claims 10-12 **characterized in that** it additionally comprises primers and probes for the amplification of cDNA of one or more reference genes among *RPL13a, GUSB, TRFC,* and/or *Beta-actin.*

14. The kit of any one of claims 10-13 **characterized in that** the quantitative analysis of the amplified cDNAs is performed by dPCR.

15. The kit of any of claims 10-14 **characterized in that** the tumor is breast cancer.

16. An *in vitro* process for monitoring the clinical status of an individual with breast cancer under treatment, **characterized in that** it comprises the steps of:
a) quantifying *LIF* expression in a liquid sample from an individual with breast cancer under treatment;
b) quantifying *LIF* expression in a liquid sample from an individual with breast cancer not under treatment;
c) comparing the quantification of *LIF* expression in the sample from the individual with breast cancer under treatment to the quantification in the sample from the individual with breast cancer not under treatment,
wherein *LIF* expression values in the sample from the individual with breast cancer under treatment that are higher than those in the sample from the individual with breast cancer not under treatment indicate an improvement in the clinical status.
